(19) Europäisches Patentamt
European Patent Office
Office européen des brevets

(11) **EP 4 299 764 A1**

(12) **EUROPEAN PATENT APPLICATION**

(43) Date of publication:
**03.01.2024  Bulletin 2024/01**

(21) Application number: **23180753.8**

(22) Date of filing: **21.06.2023**

(51) International Patent Classification (IPC):
***C12Q 1/6886*** (2018.01)

(52) Cooperative Patent Classification (CPC):
**C12Q 1/6886;** C12Q 2600/118; C12Q 2600/154

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.06.2022  US 202263356150 P**

(71) Applicant: **Universal Diagnostics, S.A.
41013 Sevilla (ES)**

(72) Inventor: **Kruusmaa, Kristi
41013 Sevilla (ES)**

(74) Representative: **Carlos Hernando, Borja
Garrigues IP, S.L.P.
Hermosilla, 3
28001 Madrid (ES)**

Remarks:
The complete document including Reference
Table(s) and the Sequence Listing(s) can be
downloaded from the EPO website

(54) **METHODS FOR DETECTING PANCREATIC CANCER USING DNA METHYLATION MARKERS**

(57)    The present disclosure provides for, among other things, methods and systems for detecting, diagnosing, predicting, monitoring, staging, and/or providing survival prognosis for pancreatic cancer using DNA methylation markers. For example, described herein are DNA methylation markers that enable categorization and prognosis evaluation of pancreatic cancer (PaCa) patients with high accuracy from human biospecimens.

EP 4 299 764 A1

**Description**

**TECHNICAL FIELD**

This invention relates generally to methods and systems for detecting, diagnosing, predicting, monitoring, staging, and/or providing survival prognosis for pancreatic cancer.

**BACKGROUND**

Pancreatic cancer (PaCa) is one of the highest mortality rate cancers worldwide with 5-year relative survival of 10.8% (GLOBOCAN 2020). According to the GLOBOCAN 2020 cancer report, the annual worldwide PaCa incidence is measured to be 56,654, causing approximately 47,700 deaths per year.

PaCa is one of the leading death-causing cancers among all cancers worldwide, due to its fast-growing nature, and due to the lack of screening programs for PaCa. Furthermore, there is a lack of guidelines for high-risk, pre-PaCa groups, and imaging methods for early diagnosis of localized stage PaCa have low sensitivity. Thus, there is an urgent need for a high-sensitivity early detection test for PaCa. It is expected that a correct risk stratification based on marker patterns in PaCa would result in reduced incidence of PaCa and decreased mortality rates.

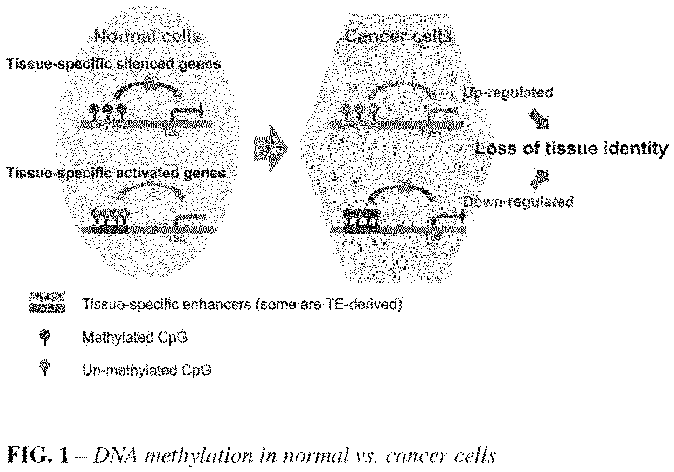

Deoxyribonucleic acid (DNA) methylation (DNAme) is an important epigenetic marker in diverse species. DNA methylation in vertebrates is characterized by the addition of a methyl or hydroxymethyl group to the C5 position of cytosine, which occurs mainly in the context of CG dinucleotides. DNA changes in methylation states is a mechanism for inactivation of cancer-associated genes, including tumor suppressor genes, in PaCa and in other human cancers. FIG. 1 shows a simplified example of how DNA methylation changes gene activation in cancer cells compared to normal cells.

DNA methylation impacts numerous cellular processes including, for example, cellular differentiation. Dysregulation of methylation, therefore, can lead to disease, including cancer. Accumulated changes in DNA methylation (e.g., hypermethylation or hypomethylation), especially when the changes are located in crucial genes, can result in cancerous cells. These changes in methylation status, if detected, can be used to predict susceptibility of a subject to developing cancer, as well as the development or presence of cancer and, potentially, other diseases.

There is a need for finding new and more efficient markers that can be used in a non-invasive and high adherence yielding PaCa screening context. Using stratification results, it would be possible to improve treatment outcomes by providing an early detection opportunity for PaCa patients.

**SUMMARY**

The present disclosure provides for, among other things, methods and systems for detecting, diagnosing, predicting, monitoring, staging, and/or providing survival prognosis for pancreatic cancer using DNA methylation markers. For example, described herein are DNA methylation markers that enable categorization and prognosis evaluation of pancreatic cancer (PaCa) patients with high accuracy from human biospecimens.

In certain embodiments, the specified markers or maker panels made up of the specified markers provide pancreatic cancer screening at high specificity at early or premalignant stage, minimizing the negative and false positive cases when interrogated in samples taken from a subject. The methylation markers specified herein demonstrate high accuracy PaCa stratification of patients whose anonymized data was obtained from The Cancer Genome Atlas (TCGA) database.

Acquired methylation events in promoter regions of tumor suppressor genes are thought to silence expression, whereas demethylation events at the promoter regions of oncogenes are thought to activate these genes, thus contributing to oncogenesis. DNA methylation is believed to be a more chemically and biologically stable process for diagnostic tool than RNA or protein expression. Furthermore, methylation events are thought to be highly tissue specific, making these markers excellent for high specificity and are more broadly informative and sensitive than are individual DNA mutations. Aberrant methylation patterns of CpG islands located in the promoter regions of tumor suppressor genes is an important mechanism for gene inactivation.

In experiments conducted while developing the biomarker panel described herein, markers were identified in PaCa samples by comparing gene expression and the methylation state of DNA markers from data obtained with Illumina 450k methylation microarrays and been collected by the TCGA consortium.

Resulting CpGs shown in Table 1 were analyzed from a total of 184 PaCa patients and healthy controls studied retrospectively, collected by the TCGA consortium. The candidate markers could provide the possibility to stratify cellular differences on the origin of the tumor and predict patient prognosis and clinical outcome. As such, the analysis provides specific markers or marker combinations for purposes of PaCa stratification and tumor origin identification. As a result, the increased levels of patient diagnosis, prognosis, clinical outcome and survival may be achieved.

*Table 1: List of top 25 genomic regions found to have significantly altered methylation pattern in PaCa patients, allowing for patient stratification and survival prediction. Each of the above DMRs is a construct of 302 bp that spans around a single differentially methylated CpG (DMC) - each DMR has a CpG at center (2 bp) with 150 bp to the right and left.*

| Symbol | Chr | start | end | SEQUENCE ID NO. |
|---|---|---|---|---|
| PTPRN; PTPRN | chr2 | 219309329 | 219309630 | 1 |
| RYR2 | chr1 | 237042549 | 237042850 | 2 |
| . | chr10 | 100659710 | 100660011 | 3 |
| GPR26 | chr10 | 123665959 | 123666260 | 4 |
| . | chr5 | 1875622 | 1875923 | 5 |
| AC003986.5; FERD3L | chr7 | 19145188 | 19145489 | 6 |
| AC010729.1; SOX11 | chr2 | 5695829 | 5696130 | 7 |
| TWIST1 | chr7 | 19106259 | 19106560 | 8 |
| RYR2 | chr1 | 237042500 | 237042801 | 9 |
| RP11-572N21.1; SP9 | chr2 | 174334950 | 174335251 | 10 |
| THBS4; THBS4 | chr5 | 80034970 | 80035271 | 11 |
| MDGA2 | chr14 | 47675647 | 47675948 | 12 |
| DBX1 | chr11 | 20156156 | 20156457 | 13 |
| . | chr7 | 35257207 | 35257508 | 14 |
| TMEM132D | chr12 | 129903539 | 129903840 | 15 |
| GDF6; GDF6 | chr8 | 96159449 | 96159750 | 16 |
| TWIST1; TWIST1 | chr7 | 19117490 | 19117791 | 17 |
| NPBWR1 | chr8 | 52939320 | 52939621 | 18 |
| . | chr13 | 112057477 | 112057778 | 19 |
| MDGA2 | chr14 | 47675755 | 47676056 | 20 |
| SORCS3; SORCS3 | chr10 | 104640972 | 104641273 | 21 |
| PEX5L; PEX5L | chr3 | 180036595 | 180036896 | 22 |
| AC009487.5; SLC4A10 | chr2 | 161427044 | 161427345 | 23 |
| . | chr20 | 56925895 | 56926196 | 24 |
| SLC6A15; SLC6A15 | chr12 | 84912868 | 84913169 | 25 |

As described with respect to the top DMRs in Table 1, may be used in identifying (e.g., sub-categorizing, stratifying, detecting, diagnosing, predicting, monitoring, staging, and/or providing survival prognosis for) pancreatic cancer.

Furthermore, in certain embodiments the marker panel could be a combination of 1600 or fewer genomic regions, or, in some embodiments, the marker is one region. In some embodiments the marker is in a high-density promoter region. In certain embodiments, the markers and/or panels of markers including methylations as well as mutations.

In one aspect, the invention is directed to a method of identifying (e.g., sub-categorization, stratification, detecting, diagnosing, predicting, monitoring, staging, and/or providing survival prognosis for) pancreatic cancer in a human subject, the method comprising: determining a methylation status of each of one or more (e.g., 1, 2, 3, 4, 5, 6, 7, 8, 9, 10, 11, 12, 13, 14, 15, 16, 17, 18, 19, 20, 21, 22, 23, 24, or 25) markers identified in deoxyribonucleic acid (DNA) from a sample obtained from the subject, and identifying (e.g., detecting, diagnosing, predicting, monitoring, staging, and/or providing survival prognosis for) pancreatic cancer in the subject based at least in part on the determined methylation status of each of the one or more markers, wherein each of the one or more markers is a methylation locus comprising at least a portion of a differentially methylated region (DMR) selected from the DMRs listed in Table 1 [or at least a single differentially methylated CpG site (DMC) within one or more of the DMRs listed in Table 1] as follows (e.g., each said portion comprising at least one (1) CpG and each said methylation locus having a length equal to or less than 302 bp): chr2:219309329-219309630 (SEQ. ID NO. 1), chr1 :237042549-237042850 (SEQ. ID NO. 2), chr10: 100659710-100660011 (SEQ. ID NO. 3), chr10: 123665959-123666260 (SEQ. ID NO. 4), chr5: 1875622-1875923 (SEQ. ID NO. 5), chr7: 19145188-19145489 (SEQ. ID NO. 6), chr2: 5695829-5696130 (SEQ. ID NO. 7), chr7: 19106259-19106560 (SEQ. ID NO. 8), chr1: 237042500 - 237042801 (SEQ. ID NO. 9), chr2: 174334950-174335251 (SEQ. ID NO. 10), chr5: 80034970-80035271 (SEQ. ID NO. 11), chr14: 47675647-47675948 (SEQ. ID NO. 12), chr11: 20156156-20156457 (SEQ. ID NO. 13), chr7: 35257207-35257508 (SEQ. ID NO. 14), chr12: 129903539-129903840 (SEQ. ID NO. 15), chr8: 96159449-96159750 (SEQ. ID NO. 16), chr7: 19117490-19117791 (SEQ. ID NO. 17), chr8: 52939320-52939621 (SEQ. ID NO. 18), chr13: 112057477-112057778 (SEQ. ID NO. 19), chr14: 47675755-47676056 (SEQ. ID NO. 20), chr10: 104640972-104641273 (SEQ. ID NO. 21), chr3: 180036595-180036896 (SEQ. ID NO. 22), chr2: 161427044-161427345 (SEQ. ID NO. 23), chr20: 56925895-56926196 (SEQ. ID NO. 24), chr12:84912868-84913169 (SEQ. ID NO. 25).

In another aspect, the invention is directed to a method of identifying (e.g., sub-categorization, stratification, detecting, diagnosing, predicting, monitoring, staging, and/or providing survival prognosis for) pancreatic cancer in a human subject, the method comprising: determining a methylation status of each of one or more markers identified in deoxyribonucleic acid (DNA) from a sample obtained from the subject, and identifying (e.g., detecting, diagnosing, predicting, monitoring, staging, and/or providing survival prognosis for) pancreatic cancer in the subject based at least in part on the determined methylation status of each of the one or more markers, wherein each of the one or more markers is a methylation locus comprising at least a portion of a differentially methylated region (DMR) selected from the DMRs listed in Table 1 [or at least a single differentially methylated CpG site (DMC) within one or more of the DMRs listed in Table 1] as follows (e.g., each said portion comprising at least one (1) CpG and each said methylation locus having a length equal to or less than 302 bp).

In certain embodiments, the sample is a tissue sample, a blood sample, a stool sample, or a blood product sample.

In certain embodiments, the sample comprises DNA that is isolated from blood or plasma of the human subject.

In certain embodiments, the DNA is cell-free DNA (cfDNA) of the human subject.

In certain embodiments, the method comprises determining the methylation status of each of the one or more markers using next generation sequencing (NGS).

In certain embodiments, method comprises using one or more capture baits that enrich for a target region to capture one or more corresponding methylation locus / loci.

In certain embodiments, each methylation locus is equal to or less than 302bp in length.

In another aspect, the invention is directed to a method comprising: converting unmethylated cytosines of a plurality of DNA fragments in a sample into uracils to generate a plurality of converted DNA fragments, wherein the plurality of DNA fragments were obtained from a biological sample; and sequencing the plurality of converted DNA fragments to generate a plurality of sequence reads, wherein each sequence read corresponds to a converted DNA fragment.

In certain embodiments, the plurality of DNA fragments (in total) comprise at least 1 ng, at least 5 ng, at least 10 ng, or at least 20 ng of DNA.

In certain embodiments, the plurality of DNA fragments consist essentially of DNA fragments each of which has a length in a range from 100bp to 600bp (e.g., from about 125bp to about 200bp, or from about 140bp to about 160bp (e.g., for cfDNA))(e.g., about 150bp to about 350bp, or from about 200bp to about 300bp (e.g., for sheared DNA)).

In certain embodiments, the plurality of DNA fragments consist essentially of DNA fragments each of which has a length in a range from 1000bp to 200,000bp [e.g., an average length of about 10,000bp (e.g., for genomic DNA, e.g., from a sample comprising tissue or buffy coat)].

In certain embodiments, each of the plurality of sequence reads are at least 50bp, at least 100bp, at least 150bp, at least 200bp, at least 300bp, or more.

## DEFINITIONS

In this application, the use of "or" means "and/or" unless stated otherwise. The articles "a" and "an" are used herein to refer to one or to more than one (*i.e.,* to at least one) of the grammatical object of the article. By way of example, "an element" refers to one element or more than one element. As used in this application, the term "comprise" and variations of the term, such as "comprising" and "comprises," are not intended to exclude other additives, components, integers or steps. As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Administration:* As used herein, the term "administration" typically refers to the administration of a composition to a subject or system, for example to achieve delivery of an agent that is, is included in, or is otherwise delivered by, the composition. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intravenous, intraventricular, within a specific organ (e. g. intrahepatic), mucosal, nasal, oral, rectal, subcutaneous, sublingual, topical, tracheal (including by intratracheal instillation), transdermal, vaginal and vitreal. In some embodiments, administration may involve intermittent dosing. In some embodiments, administration may involve continuous dosing (e.g., perfusion) for at least a selected period of time. As is known in the art, antibody therapy is commonly administered parenterally (e.g., by intravenous or subcutaneous injection).

*Agent*: As used herein, the term "agent" refers to an entity (e.g., for example, a small molecule, peptide, polypeptide, nucleic acid, lipid, polysaccharide, complex, combination, mixture, system, or phenomenon such as heat, electric current, electric field, magnetic force, magnetic field, etc.).

*Amelioration:* As used herein, the term "amelioration" refers to the prevention, reduction, palliation, or improvement of a state of a subject. Amelioration includes, but does not require, complete recovery or complete prevention of a disease, disorder or condition.

*Amplicon or amplicon molecule:* As used herein, the term "amplicon" or "amplicon molecule" refers to a nucleic acid molecule generated by transcription from a template nucleic acid molecule, or a nucleic acid molecule having a sequence complementary thereto, or a double-stranded nucleic acid including any such nucleic acid molecule. Transcription can be initiated from a primer.

*Amplification:* As used herein, the term "amplification" refers to the use of a template nucleic acid molecule in combination with various reagents to generate further nucleic acid molecules from the template nucleic acid molecule, which further nucleic acid molecules may be identical to or similar to (e.g., at least 70% identical, e.g., at least 75%, 80%, 85%, 90%, 91%, 92%, 93%, 94%, 95%, 96%, 97%, 98%, 99%, or 100% identical to) a segment of the template nucleic acid molecule and/or a sequence complementary thereto. Amplification generally refers to the production of multiple copies of a specific nucleic acid portion, typically starting from a small amount of the polynucleotide. It is to be differentiated from non-specific template replication (e.g., replication that is template-dependent but not dependent on a specific template). Template specificity is frequently described in terms of "target" specificity. Target sequences are "targets" in the sense that they are sought to be sorted out from other nucleic acid. Amplification techniques, like, but not limited to, polymerase-chain reaction (PCR), have been designed primarily for this sorting out.

*Amplification reaction mixture:* As used herein, the terms "amplification reaction mixture" or "amplification reaction" refer to a template nucleic acid molecule together with reagents sufficient for amplification of the template nucleic acid molecule.

*Biological pathway*: As used herein, the term "biological pathway" refers to a set of interactions or changes in a cell that leads to production of a specific product or a change in cell fate. Each pathway in combination with other pathways or separately can cause a particular molecule production, switch gene activity or lead the cell to a particular position, etc. Herein, these set of interactions are used to demonstrate the relationship between the aberrant methylated regions and its biological function, determining its clinical and diagnostic values.

*Biological Sample*: As used herein, the term "biological sample" generally refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell- containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semi- permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

*Biomarker*: As used herein, the term "biomarker," consistent with its use in the art, refers to a to an entity whose presence, level, or form, correlates with a particular biological event or state of interest, so that it is considered to be a "marker" of that event or state. Those of skill in the art will appreciate, for instance, in the context of a DNA biomarker, that a biomarker can be or include a locus (such as one or more methylation loci) and/or the status of a locus (e.g., the status of one or more methylation loci). To give but a few examples of biomarkers, in some embodiments, e.g., as set forth herein, a biomarker can be or include a marker for a particular disease, disorder or condition, or can be a marker for qualitative of quantitative probability that a particular disease, disorder or condition can develop, occur, or reoccur, e.g., in a subject. In some embodiments, e.g., as set forth herein, a biomarker can be or include a marker for a particular therapeutic outcome, or qualitative of quantitative probability thereof. Thus, in various embodiments, e.g., as set forth herein, a biomarker can be predictive, prognostic, and/or diagnostic, of the relevant biological event or state of interest. A biomarker can be an entity of any chemical class. For example, in some embodiments, e.g., as set forth herein, a biomarker can be or include a nucleic acid, a polypeptide, a lipid, a carbohydrate, a small molecule, an inorganic agent (e.g., a metal or ion), or a combination thereof. In some embodiments, e.g., as set forth herein, a biomarker is a cell surface marker. In some embodiments, e.g., as set forth herein, a biomarker is intracellular. In some embodiments, e.g., as set forth herein, a biomarker is found outside of cells (e.g., is secreted or is otherwise generated or present outside of cells, e.g., in a body fluid such as blood, urine, tears, saliva, cerebrospinal fluid, and the like). In some embodiments, e.g., as set forth herein, a biomarker is methylation status of a methylation locus. In some instances, e.g., as set forth herein, a biomarker may be referred to as a "marker."

To give but one example of a biomarker, in some embodiments e.g., as set forth herein, the term refers to expression of a product encoded by a gene, expression of which is characteristic of a particular tumor, tumor subclass, stage of tumor, etc. Alternatively or additionally, in some embodiments, e.g., as set forth herein, presence or level of a particular marker can correlate with activity (or activity level) of a particular signaling pathway, for example, of a signaling pathway the activity of which is characteristic of a particular class of tumors.

Those of skill in the art will appreciate that a biomarker may be individually determinative of a particular biological event or state of interest, or may represent or contribute to a determination of the statistical probability of a particular biological event or state of interest. Those of skill in the art will appreciate that markers may differ in their specificity and/or sensitivity as related to a particular biological event or state of interest.

*Biomolecule*: As used herein, "biomolecule" refers to bioactive, diagnostic, and prophylactic molecules. Biomolecules that can be used in the present invention include, but are not limited to, synthetic, recombinant or isolated peptides and proteins such as antibodies and antigens, receptor ligands, enzymes, and adhesion peptides; nucleotides and polynucleic acids such as DNA and antisense nucleic acid molecule; activated sugars and polysaccharides; bacteria; viruses; and chemical drugs such as antibiotics, anti-inflammatories, and antifungal agents.

*Bisulfite reagent:* As used herein, the term "bisulfite reagent refers to a reagent comprising bisulfite, disulfite, hydrogen sulfite, or combinations thereof to distinguish between methylated and unmethylated cytidines, e.g., in CpG dinucleotide sequences. *Blood component:* As used herein, the term "blood component" refers to any component of whole blood, including red blood cells, white blood cells, plasma, platelets, endothelial cells, mesothelial cells, epithelial cells, and cell-free DNA. Blood components also include the components of plasma, including proteins, metabolites, lipids, nucleic acids, and carbohydrates, and any other cells that can be present in blood, e.g., due to pregnancy, organ transplant, infection, injury, or disease.

*Cancer*: As used herein, the terms "cancer," "malignancy," "neoplasm," "tumor," and "carcinoma," are used interchangeably to refer to a disease, disorder, or condition in which cells exhibit or exhibited relatively abnormal, uncontrolled, and/or autonomous growth, so that they display or displayed an abnormally elevated proliferation rate and/or aberrant growth phenotype. In some embodiments, e.g., as set forth herein, a cancer can include one or more tumors. In some embodiments e.g., as set forth herein, a cancer can be or include cells that are precancerous (e.g., benign), malignant, pre-metastatic, metastatic, and/or non-metastatic. In some embodiments e.g., as set forth herein, a cancer can be or include a solid tumor. In some embodiments e.g., as set forth herein, a cancer can be or include a hematologic tumor. In general, examples of different types of cancers known in the art include, for example, colorectal cancer, hematopoietic cancers including leukemias, lymphomas (Hodgkin's and non-Hodgkin's), myelomas and myeloproliferative disorders; sarcomas, melanomas, adenomas, carcinomas of solid tissue, squamous cell carcinomas of the mouth, throat, larynx, and lung, liver cancer, genitourinary cancers such as prostate, cervical, bladder, uterine, and endometrial cancer and renal cell carcinomas, bone cancer, pancreatic cancer, skin cancer, cutaneous or intraocular melanoma, cancer of the endocrine system, cancer of the thyroid gland, cancer of the parathyroid gland, head and neck cancers, breast cancer, gastro-intestinal cancers and nervous system cancers, benign lesions such as papillomas, and the like.

*Chemotherapeutic agent*: As used herein, the term "chemotherapeutic agent," consistent with its use in the art, refers to one or more agents known, or having characteristics known to, treat or contribute to the treatment of cancer.

In particular, chemotherapeutic agents include pro-apoptotic, cytostatic, and/or cytotoxic agents. In some embodiments e.g., as set forth herein, a chemotherapeutic agent can be or include alkylating agents, anthracyclines, cytoskeletal disruptors (e.g., microtubule targeting moieties such as taxanes, maytansine, and analogs thereof, of), epothilones, histone deacetylase inhibitors HDACs), topoisomerase inhibitors (e.g., inhibitors of topoisomerase I and/or topoisomerase II), kinase inhibitors, nucleotide analogs or nucleotide precursor analogs, peptide antibiotics, platinum-based agents, retinoids, vinca alkaloids, and/or analogs that share a relevant anti-proliferative activity. In some particular embodiments e.g., as set forth herein, a chemotherapeutic agent can be or include of Actinomycin, All-trans retinoic acid, an Auiristatin, Azacitidine, Azathioprine, Bleomycin, Bortezomib, Carboplatin, Capecitabine, Cisplatin, Chlorambucil, Cyclophosphamide, Curcumin, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Maytansine and/or analogs thereof (e.g., DM1) Mechlorethamine, Mercaptopurine, Methotrexate, Mitoxantrone, a Maytansinoid, Oxaliplatin, Paclitaxel, Pemetrexed, Teniposide, Tioguanine, Topotecan, Valrubicin, Vinblastine, Vincristine, Vindesine, Vinorelbine, or a combination thereof. In some embodiments e.g., as set forth herein, a chemotherapeutic agent can be utilized in the context of an antibody-drug conjugate. In some embodiments e.g., as set forth herein, a chemotherapeutic agent is one found in an antibody-drug conjugate selected from the group consisting of: hLL1-doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicin, gemtuzumab ozogamicin, brentuximab vedotin, trastuzumab emtansine, inotuzumab ozogamicin, glembatumomab vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, vorsetuzumab mafodotin, and lorvotuzumab mertansine. In some embodiments e.g., as set forth herein, a chemotherapeutic agent can be or comprise of farnesyl-thiosalicylic acid (FTS), 4-(4-Chloro-2-methylphenoxy)-N-hydroxybutanamide (CMH), estradiol (E2), tetramethoxystilbene (TMS), δ-tocatrienol, salinomycin, or curcumin.

*Combination therapy:* As used herein, the term "combination therapy" refers to administration to a subject of to two or more agents or regimens such that the two or more agents or regimens together treat a disease, condition, or disorder of the subject. In some embodiments, e.g., as set forth herein, the two or more therapeutic agents or regimens can be administered simultaneously, sequentially, or in overlapping dosing regimens. Those of skill in the art will appreciate that combination therapy includes but does not require that the two agents or regimens be administered together in a single composition, nor at the same time.

*Comparable:* As used herein, the term "comparable" refers to members within sets of two or more conditions, circumstances, agents, entities, populations, etc., that may not be identical to one another but that are sufficiently similar to permit comparison there between, such that one of skill in the art will appreciate that conclusions can reasonably be drawn based on differences or similarities observed. In some embodiments, e.g., as sort forth herein, comparable sets of conditions, circumstances, agents, entities, populations, etc. are typically characterized by a plurality of substantially identical features and zero, one, or a plurality of differing features. Those of ordinary skill in the art will understand, in context, what degree of identity is required to render members of a set comparable. For example, those of ordinary skill in the art will appreciate that members of sets of conditions, circumstances, agents, entities, populations, etc., are comparable to one another when characterized by a sufficient number and type of substantially identical features to warrant a reasonable conclusion that differences observed can be attributed in whole or part to non-identical features thereof.

*Corresponding to:* As used herein, the term "corresponding to" refers to a relationship between two or more entities. For example, the term "corresponding to" may be used to designate the position/identity of a structural element in a compound or composition relative to another compound or composition (e.g., to an appropriate reference compound or composition). For example, in some embodiments, a monomeric residue in a polymer (e.g., a nucleic acid residue in a polynucleotide) may be identified as "corresponding to" a residue in an appropriate reference polymer. Those of ordinary skill in the art readily appreciate how to identify "*corresponding*" nucleic acids. For example, those skilled in the art will be aware of various sequence alignment strategies, including software programs such as, for example, BLAST, CS-BLAST, CUSASW++, DIAMOND, FASTA, GGSEARCH/GLSEARCH, Genoogle, HMMER, HHpred/HHsearch, IDF, Infernal, KLAST, USEARCH, parasail, PSI-BLAST, PSI-Search, ScalaBLAST, Sequilab, SAM, SSEARCH, SWAPHI, SWAPHI-LS, SWIMM, or SWIPE that can be utilized, for example, to identify "corresponding" residues in nucleic acids in accordance with the present disclosure. Those of skill in the art will also appreciate that, in some instances, the term "corresponding to" may be used to describe an event or entity that shares a relevant similarity with another event or entity (e.g., an appropriate reference event or entity). To give but one example, a fragment of DNA in a sample from a subject may be described as "corresponding to" a gene in order to indicate, in some embodiments, that it shows a particular degree of sequence identity or homology, or shares a particular characteristic sequence element.

*Detectable moiety:* The term "detectable moiety" as used herein refers to any element, molecule, functional group, compound, fragment, or other moiety that is detectable. In some embodiments, e.g., as sort forth herein, a detectable moiety is provided or utilized alone. In some embodiments, e.g., as sort forth herein, a detectable moiety is provided and/or utilized in association with (e.g., joined to) another agent. Examples of detectable moieties include, but are not limited to, various ligands, radionuclides (e.g., $^3$H, $^{14}$C, $^{18}$F, $^{19}$F, $^{32}$P, $^{35}$S, $^{135}$I, $^{125}$I, $^{123}$I, $^{64}$Cu, $^{187}$Re, $^{111}$In, $^{90}$Y, $^{99m}$Tc, $^{177}$Lu, $^{89}$Zr etc.), fluorescent dyes, chemiluminescent agents, bioluminescent agents, spectrally resolvable inorganic fluorescent semiconductors nanocrystals (i.e., quantum dots), metal nanoparticles, nanoclusters, paramagnetic metal ions, enzymes, colorimetric labels, biotin, dioxigenin, haptens, and proteins for which antisera or monoclonal antibodies are available.

*Diagnosis*: As used herein, the term "Diagnosis" refers to determining whether, and/or the qualitative of quantitative probability that, a subject has or will develop a disease, disorder, condition, or state. For example, in diagnosis of cancer, diagnosis can include a determination regarding the risk, type, stage, malignancy, or other classification of a cancer. In some instances, e.g., as sort forth herein, a diagnosis can be or include a determination relating to prognosis and/or likely response to one or more general or particular therapeutic agents or regimens.

*Diagnostic information*: As used herein, the term "diagnostic information" or "information for use in diagnosis" refers to information useful in determining whether a patient has a disease, disorder or condition and/or in classifying a disease, disorder or condition into a phenotypic category or any category having significance with regard to prognosis of a disease, disorder or condition, or likely response to treatment (either treatment in general or any particular treatment) of a disease, disorder or condition. Similarly, "diagnosis" refers to providing any type of diagnostic information, including, but not limited to, whether a subject is likely to have or develop a disease, disorder or condition, state, staging or characteristic of a disease, disorder or condition as manifested in the subject, information related to the nature or classification of a tumor, information related to prognosis and/or information useful in selecting an appropriate treatment. Selection of treatment may include the choice of a particular therapeutic agent or other treatment modality such as surgery, radiation, etc., a choice about whether to withhold or deliver therapy, a choice relating to dosing regimen (e.g., frequency or level of one or more doses of a particular therapeutic agent or combination of therapeutic agents), etc. Diagnostic information can include, without limitation, biomarker status information.

*Differentially methylated*: As used herein, the term "differentially methylated" describes a methylation site for which the methylation status differs between a first condition and a second condition. A methylation site that is differentially methylated can be referred to as a differentially methylated site. In some instances, e.g., as sort forth herein, a DMR is defined by the amplicon produced by amplification using oligonucleotide primers , e.g., a pair of oligonucleotide primers selected for amplification of the DMR or for amplification of a DNA region of interest present in the amplicon. In some instances, e.g., as sort forth herein, a DMR is defined as a DNA region amplified by a pair of oligonucleotide primers, including the region having the sequence of, or a sequence complementary to, the oligonucleotide primers. In some instances, e.g., as sort forth herein, a DMR is defined as a DNA region amplified by a pair of oligonucleotide primers, excluding the region having the sequence of, or a sequence complementary to, the oligonucleotide primers. As used herein, a specifically provided DMR can be unambiguously identified by the name of an associated gene followed by three digits of a starting position, such that, for example, a DMR starting at position 100785927of ZAN can be identified as ZAN '927. As used herein, a specifically provided DMR can be unambiguously identified by the chromosome number followed by the starting and ending positions of a DMR.

*Differentially methylated region*: As used herein, the term "differentially methylated region" (DMR) refers to a DNA region that includes one or more differentially methylated sites. A DMR that includes a greater number or frequency of methylated sites under a selected condition of interest, such as a cancerous state, can be referred to as a hypermethylated DMR. A DMR that includes a smaller number or frequency of methylated sites under a selected condition of interest, such as a cancerous state, can be referred to as a hypomethylated DMR. A DMR that is a methylation biomarker for pancreatic cancer can be referred to as a pancreatic cancer DMR. A DMR that is a methylation biomarker for colorectal cancer can be referred to as a colorectal cancer DMR. A DMR that is a methylation biomarker for advanced adenoma can be referred to as an advanced adenoma DMR. In some instances, e.g., as set forth herein, a DMR can be a single nucleotide, which single nucleotide is a methylation site. In some instances, e.g., as set forth herein, a DMR has a length of at least 10, at least 15, at least 20, at least 30, at least 50, or at least 75 base pairs. In some instances, e.g., as set forth herein, a DMR has a length of equal to or less than 5000 bp, 4,000 bp, 3,000 bp, 2,000 bp, 1,000 bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp (e.g., where methylation status is determined using quantitative polymerase chain reaction (qPCR), e.g., methylation sensitive restriction enzyme quantitative polymerase chain reaction (MSRE-qPCR)) (e.g., where methylation status is determined using a next generation sequencing technique, e.g., targeted next generation sequencing).

*DNA region*: As used herein, "DNA region" refers to any contiguous portion of a larger DNA molecule. Those of skill in the art will be familiar with techniques for determining whether a first DNA region and a second DNA region correspond, based, e.g., on sequence similarity (e.g, sequence identity or homology) of the first and second DNA regions and/or context (e.g., the sequence identity or homology of nucleic acids upstream and/or downstream of the first and second DNA regions).

Except as otherwise specified herein, sequences found in or relating to humans (e.g., that hybridize to human DNA) are found in, based on, and/or derived from the example representative human genome sequence commonly referred to, and known to those of skill in the art, as Homo sapiens (human) genome assembly GRCh38, hg38, and/or Genome Reference Consortium Human Build 38. Those of skill in the art will further appreciate that DNA regions of hg38 can be referred to by a known system including identification of particular nucleotide positions or ranges thereof in accordance with assigned numbering.

*Dosing regimen:* As used herein, the term "dosing regimen" can refer to a set of one or more same or different unit doses administered to a subject, typically including a plurality of unit doses administration of each of which is separated from administration of the others by a period of time. In various embodiments, e.g., as set forth herein, one or more or all unit doses of a dosing regimen may be the same or can vary (e.g., increase over time, decrease over time, or be adjusted in accordance with the subject and/or with a medical practitioner's determination). In various embodiments, e.g., as set forth herein, one or more or all of the periods of time between each dose may be the same or can vary (e.g., increase over time, decrease over time, or be adjusted in accordance with the subject and/or with a medical practitioner's determination). In some embodiments, e.g., as set forth herein, a given therapeutic agent has a recommended dosing regimen, which can involve one or more doses. Typically, at least one recommended dosing regimen of a marketed drug is known to those of skill in the art. In some embodiments, e.g., as set forth herein, a dosing regimen is correlated with a desired or beneficial outcome when administered across a relevant population (i.e., is a therapeutic dosing regimen).

*Downstream:* As used herein, the term" downstream" means that a first DNA region is closer, relative to a second DNA region, to the C-terminus of a nucleic acid that includes the first DNA region and the second DNA region.

*Early stage:* As used herein, the term "early stage" refers to a localized stage where cancer has not yet spread to nearby lymph nodes (N0) or to distant sites (M0). For example, pathologically it would be cancer stages from stage 0 to stage II C.

*Gene*: As used herein, the term "gene" refers to a single DNA region, e.g., in a chromosome, that includes a coding sequence that encodes a product (e.g., an RNA product and/or a polypeptide product), together with all, some, or none of the DNA sequences that contribute to regulation of the expression of coding sequence. In some embodiments, e.g., as set forth herein, a gene includes one or more non-coding sequences. In some particular embodiments, e.g., as set forth herein, a gene includes exonic and intronic sequences. In some embodiments, e.g., as set forth herein, a gene includes one or more regulatory elements that, for example, can control or impact one or more aspects of gene expression (e.g., cell-type-specific expression, inducible expression, etc.). In some embodiments, e.g., as set forth herein, a gene includes a promoter. In some embodiments, e.g., as set forth herein, a gene includes one or both of a (i) DNA nucleotides extending a predetermined number of nucleotides upstream of the coding sequence and (ii) DNA nucleotides extending a predetermined number of nucleotides downstream of the coding sequence. In various embodiments, e.g., as set forth herein, the predetermined number of nucleotides can be 500 bp, 1 kb, 2 kb, 3 kb, 4 kb, 5 kb, 10 kb, 20 kb, 30 kb, 40 kb, 50 kb, 75 kb, or 100 kb.

*Gene ontology*: The term "gene ontology" (GO) refers to a set of concepts to describe the function of gene products in organisms. It represents computational aspects of biological systems. GO has independent categories, herein are used two: biological process and molecular function, in each category gene products in different biological system concepts are analyzed. These concepts are used to demonstrate the relationship between the aberrant methylated regions and its biological function, determining its clinical and diagnostic values.

*Homology*: As used herein, the term "homology" refers to the overall relatedness between polymeric molecules, *e.g.,* between nucleic acid molecules (*e.g.,* DNA molecules and/or RNA molecules) and/or between polypeptide molecules. Those of skill in the art will appreciate that homology can be defined, e.g., by a percent identity or by a percent homology (sequence similarity). In some embodiments, e.g., as set forth herein, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, e.g., as set forth herein, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.

*Hybridize*: As used herein, "hybridize" refers to the association of a first nucleic acid with a second nucleic acid to form a double-stranded structure, which association occurs through complementary pairing of nucleotides. Those of skill in the art will recognize that complementary sequences, among others, can hybridize. In various embodiments, e.g., as set forth herein, hybridization can occur, for example, between nucleotide sequences having at least 70% complementarity, e.g., at least 75%, 80%, 85%, 90%, 91%, 92%, 93%, 94%, 95%, 96%, 97%, 98%, 99%, or 100% complementarity. Those of skill in the art will further appreciate that whether hybridization of a first nucleic acid and a second nucleic acid does or does not occur can dependence upon various reaction conditions. Conditions under which hybridization can occur are known in the art.

*Hypomethylation*: As used herein, the term "hypomethylation" refers to the state of a methylation locus having at least one fewer methylated nucleotides in a state of interest as compared to a reference state (e.g., at least one fewer methylated nucleotides in pancreatic cancer than in a healthy control).

*Hypermethylation*: As used herein, the term "hypermethylation" refers to the state of a methylation locus having at least one more methylated nucleotide in a state of interest as compared to a reference state (e.g., at least one more methylated nucleotide in pancreatic cancer than in a healthy control).

*Identity, identical:* As used herein, the terms "identity" and "identical" refers to the overall relatedness between polymeric molecules, *e.g.,* between nucleic acid molecules (*e.g.,* DNA molecules and/or RNA molecules) and/or between polypeptide molecules. Methods for the calculation of a percent identity as between two provided sequences are known in the art. Calculation of the percent identity of two nucleic acid or polypeptide sequences, for example, can be performed by aligning the two sequences (or the complement of one or both sequences) for optimal comparison purposes (*e.g.,* gaps can be introduced in one or both of a first and a second sequences for optimal alignment and non-identical sequences can be disregarded for comparison purposes). The nucleotides or amino acids at corresponding positions are then compared. When a position in the first sequence is occupied by the same residue (e.g., nucleotide or amino acid) as the corresponding position in the second sequence, then the molecules are identical at that position. The percent identity between the two sequences is a function of the number of identical positions shared by the sequences and, optionally, taking into account the number of gaps and the length of each gap, which may need to be introduced for optimal alignment of the two sequences. The comparison of sequences and determination of percent identity between two sequences can be accomplished using a computational algorithm, such as BLAST (basic local alignment search tool).

*"Improved," "increased," or "reduced"*: As used herein, these terms, or grammatically comparable comparative terms, indicate values that are relative to a comparable reference measurement. For example, in some embodiments, e.g., as set forth herein, an assessed value achieved with an agent of interest may be "improved" relative to that obtained with a comparable reference agent or with no agent. Alternatively or additionally, in some embodiments, e.g., as set forth herein, an assessed value in a subject or system of interest may be "improved" relative to that obtained in the same subject or system under different conditions or at a different point in time (e.g., prior to or after an event such as administration of an agent of interest), or in a different, comparable subject (e.g., in a comparable subject or system that differs from the subject or system of interest in presence of one or more indicators of a particular disease, disorder or condition of interest, or in prior exposure to a condition or agent, etc.). In some embodiments, e.g., as set forth herein, comparative terms refer to statistically relevant differences (e.g., differences of a prevalence and/or magnitude sufficient to achieve statistical relevance). Those of skill in the art will be aware, or will readily be able to determine, in a given context, a degree and/or prevalence of difference that is required or sufficient to achieve such statistical significance.

*Kaplan-Meier estimate* or *product limit estimate refers* to the statistical test with which the occurrence probability of an event at a certain time point can be measured. The probability at a certain time will be multiplied by previous possibilities to gain the results, herein is used to estimate the survival function from lifetime data.

*Locus of* a nucleic acid refers to a subregion of a nucleic acid, e.g., a CpG island, single nucleotide, a gene on a chromosome, etc.

*Marker:* A marker, as used herein, refers to an entity or moiety whose presence or level is a characteristic of a particular state or event. In some embodiments, presence or level of a particular marker may be characteristic of presence or stage of a disease, disorder, or condition. To give but one example, in some embodiments, the term refers to a gene expression product that is characteristic of a particular tumor, tumor subclass, stage of tumor, etc. Alternatively, or additionally, in some embodiments, a presence or level of a particular marker correlates with activity (or activity level) of a particular signaling pathway, for example that may be characteristic of a particular class of tumors. The statistical significance of the presence or absence of a marker may vary depending upon the particular marker. In some embodiments, detection of a marker is highly specific in that it reflects a high probability that the tumor is of a particular subclass. Such specificity may come at the cost of sensitivity (i.e., a negative result may occur even if the tumor is a tumor that would be expected to express the marker).

Conversely, markers with a high degree of sensitivity may be less specific that those with lower sensitivity. According to the present invention a useful marker need not distinguish tumors of a particular subclass with 100% accuracy. A marker may be a metabolite, lipid, fatty acid, and/or polyunsaturated fatty acid. In certain embodiments, the term marker may refer to a ratio of two entities (e.g., moieties).

*Methylation*: As used herein, the term "methylation" includes methylation at any of (i) C5 position of cytosine; (ii) N4 position of cytosine; and (iii) the N6 position of adenine. Methylation also includes (iv) other types of nucleotide methylation. A nucleotide that is methylated can be referred to as a "methylated nucleotide" or "methylated nucleotide base." Accordingly, a "methylated nucleotide" or a "methylated nucleotide base" refers to the presence of a methyl moiety on a nucleotide base, where the methyl moiety is not present in a recognized typical nucleotide base. In certain embodiments, e.g., as set forth herein, methylation specifically refers to methylation of cytosine residues. In some instances, methylation specifically refers to methylation of cytosine residues present in CpG sites.

*Methylation assay*: As used herein, the term "methylation assay" refers to any technique that can be used to determine the methylation status of a methylation locus. For example, a methylation assay may refer to a technique for determining the methylation state of one or more CpG dinucleotide sequences within a sequence of a nucleic acid.

*Methylation biomarker:* As used herein, the term "methylation biomarker" refers to a biomarker that is or includes at least one methylation locus and/or the methylation status of at least one methylation locus, e.g., a hypermethylated locus. In particular, a methylation biomarker is a biomarker characterized by a change between a first state and a second state (e.g., between a cancerous state and a non-cancerous state) in methylation status of one or more nucleic acid loci.

*Methylation locus*: As used herein, the term "methylation locus" refers to a DNA region that includes at least one differentially methylated region. A methylation locus that includes a greater number or frequency of methylated sites under a selected condition of interest, such as a cancerous state, can be referred to as a hypermethylated locus. A methylation locus that includes a smaller number or frequency of methylated sites under a selected condition of interest, such as a cancerous state, can be referred to as a hypomethylated locus. In some instances, e.g., as set forth herein, a methylation locus has a length of at least 10, at least 15, at least 20, at least 30, at least 50, or at least 75 base pairs. In some instances, e.g., as set forth herein, a methylation locus has a length of less than 5000 bp, 4,000 bp, 3,000 bp, 2,000 bp, 1,000 bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp (e.g., where methylation status is determined using quantitative polymerase chain reaction (qPCR), e.g., methylation sensitive restriction enzyme quantitative polymerase chain reaction (MSRE-qPCR)).

*Methylation site*: As used herein, a methylation site refers to a nucleotide or nucleotide position that is methylated in at least one condition. In its methylated state, a methylation site can be referred to as a methylated site.

The terms "*methylation-specific restriction enzyme*" or "*methylation-sensitive restriction enzyme*" refers to an enzyme that selectively digests a nucleic acid dependent on the methylation state of its recognition site.

*Methylation status*: As used herein, "methylation status," "methylation state," or "methylation profile" refer to the number, frequency, or pattern of methylation at methylation sites within a methylation locus. Accordingly, a change in methylation status between a first state and a second state can be or include an increase in the number, frequency, or pattern of methylated sites, or can be or include a decrease in the number, frequency, or pattern of methylated sites. In various instances, a change in methylation status in a change in methylation value.

*Methylation value*: As used herein, the term "methylation value" refers to a numerical representation of a methylation status, e.g., in the form of number that represents the frequency or ratio of methylation of a methylation locus. In some instances, e.g., as set forth herein, a methylation value can be generated by a method that includes quantifying the amount of intact nucleic acid present in a sample following restriction digestion of the sample with a methylation dependent restriction enzyme. In some instances, e.g., as set forth herein, a methylation value can be generated by a method that includes comparing amplification profiles after bisulfite reaction of a sample. In some instances, e.g., as set forth herein, a methylation value can be generated by comparing sequences of bisulfite-treated and untreated nucleic acids. In some instances, e.g., as set forth herein, a methylation value is, includes, or is based on a quantitative PCR result.

The methylation state of a particular nucleic acid sequence (e.g., a gene marker or DNA region as described herein) can indicate the methylation state of every base in the sequence or can indicate the methylation state of a subset of the bases (e.g., of one or more cytosines) within the sequence, or can indicate information regarding regional methylation density within the sequence with or without providing precise information of the locations within the sequence the methylation occurs.

The terms "methylation state", "methylation profile", and "methylation status" also refer to the relative concentration, absolute concentration, or pattern of methylated C or unmethylated C throughout any particular region of a nucleic acid in a biological sample. For example, if the cytosine (C) residue(s) within a nucleic acid sequence are methylated it may be referred to as "hypermethylated" or having "increased methylation", whereas if the cytosine (C) residue(s) within a DNA sequence are not methylated it may be referred to as "hypomethylated" or having "decreased methylation". Likewise, if the cytosine (C) residue(s) within a nucleic acid sequence are methylated as compared to another nucleic acid sequence (e.g., from a different region or from a different individual, etc.) that sequence is considered hypermethylated or having increased methylation compared to the other nucleic acid sequence. Alternatively, if the cytosine (C) residue(s) within a DNA sequence are not methylated as compared to another nucleic acid sequence (e.g., from a different region or from a different individual, etc.) that sequence is considered hypomethylated or having decreased methylation compared to the other nucleic acid sequence.

Sequences are said to be "differentially methylated" or as having a "difference in methylation" or having a "different methylation state" when they differ in the extent (e.g., one has increased or decreased methylation relative to the other), frequency, or pattern of methylation. The term "differential methylation" can refer to a difference in the level or pattern of nucleic acid methylation in a cancer positive sample as compared with the level or pattern of nucleic acid methylation in a cancer negative sample.

*Mutation:* As used herein, the term "mutation" refers to a genetic variation in a biomolecule (e.g., a nucleic acid or a protein) as compared to a reference biomolecule. For example, a mutation in a nucleic acid may, in some embodiments, comprise a nucleobase substitution, a deletion of one or more nucleobases, an insertion of one or more nucleobases, an inversion of two or more nucleobases, or a truncation, as compared to a reference nucleic acid molecule.

Similarly, a mutation in a protein may comprise an amino acid substitution, insertion, inversion, or truncation, as compared to a reference polypeptide. Additional mutations, *e.g.,* fusions and indels, are known to those of skill in the art. In some embodiments, a mutation comprises a genetic variant that is associated with a loss of function of a gene product. A loss of function may be a complete abolishment of function, e.g., an abolishment of the enzymatic activity of an enzyme, or a partial loss of function, e.g., a diminished enzymatic activity of an enzyme. In some embodiments, a mutant comprises a genetic variant that is associated with a gain of function, *e.g.,* with a negative or undesirable alteration in a characteristic or activity in a gene product. In some embodiments, a mutant is characterized by a reduction or loss in a desirable level or activity as compared to a reference; in some embodiments, a mutant is characterized by an increase or gain of an undesirable level or activity as compared to a reference. In some embodiments, the reference biomolecule is a wild-type biomolecule.

*Nucleic acid*: As used herein, in its broadest sense, the term "nucleic acid" refers to any compound and/or substance that is or can be incorporated into an oligonucleotide chain. In some embodiments e.g., as set forth herein, a nucleic acid is a compound and/or substance that is or can be incorporated into an oligonucleotide chain via a phosphodiester linkage. As will be clear from context, in some embodiments e.g., as set forth herein, the term nucleic acid refers to an individual nucleic acid residue (e.g., a nucleotide and/or nucleoside), and in some embodiments e.g., as set forth herein refers to an polynucleotide chain comprising a plurality of individual nucleic acid residues. A nucleic acid can be or include DNA, RNA, or a combinations thereof. A nucleic acid can include natural nucleic acid residues, nucleic acid analogs, and/or synthetic residues. In some embodiments e.g., as set forth herein, a nucleic acid includes natural nucleotides (e.g., adenosine, thymidine, guanosine, cytidine, uridine, deoxyadenosine, deoxythymidine, deoxy guanosine, and deoxycytidine). In some embodiments e.g., as set forth herein, a nucleic acid is or includes of one or more nucleotide analogs (e.g., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof).

In some embodiments e.g., as set forth herein, a nucleic acid has a nucleotide sequence that encodes a functional gene product such as an RNA or protein. In some embodiments e.g., as set forth herein, a nucleic acid includes one or more introns. In some embodiments e.g., as set forth herein, a nucleic acid includes one or more genes. In some embodiments e.g., as set forth herein, nucleic acids are prepared by one or more of isolation from a natural source, enzymatic synthesis by polymerization based on a complementary template (*in vivo* or *in vitro),* reproduction in a recombinant cell or system, and chemical synthesis.

In some embodiments e.g., as set forth herein, a nucleic acid analog differs from a nucleic acid in that it does not utilize a phosphodiester backbone. For example, in some embodiments e.g., as set forth herein, a nucleic acid can include one or more peptide nucleic acids, which are known in the art and have peptide bonds instead of phosphodiester bonds in the backbone. Alternatively or additionally, in some embodiments e.g., as set forth herein, a nucleic acid has one or more phosphorothioate and/or 5'-N-phosphoramidite linkages rather than phosphodiester bonds. In some embodiments e.g., as set forth herein, a nucleic acid comprises one or more modified sugars (e.g., 2'-fluororibose, ribose, 2'-deoxyribose, arabinose, and hexose) as compared with those in natural nucleic acids.

In some embodiments, e.g., as set forth herein, a nucleic acid is or includes at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues. In some embodiments, e.g., as set forth herein, a nucleic acid is partly or wholly single stranded, or partly or wholly double stranded.

*Nucleic acid detection assay*: As used herein, the term "nucleic acid detection assay" refers to any method of determining the nucleotide composition of a nucleic acid of interest. Nucleic acid detection assays include but are not limited to, DNA sequencing methods (e.g., next generation sequencing methods), polymerase chain reaction-based methods, probe hybridization methods, ligase chain reaction, etc.

*Nucleotide:* As used herein, the term "nucleotide" refers to a structural component, or building block, of polynucleotides, e.g., of DNA and/or RNA polymers. A nucleotide includes of a base (e.g., adenine, thymine, uracil, guanine, or cytosine) and a molecule of sugar and at least one phosphate group. As used herein, a nucleotide can be a methylated nucleotide or an un-methylated nucleotide. Those of skill in the art will appreciate that nucleic acid terminology, such as, as examples, "locus" or "nucleotide" can refer to both a locus or nucleotide of a single nucleic acid molecule and/or to the cumulative population of loci or nucleotides within a plurality of nucleic acids (e.g., a plurality of nucleic acids in a sample and/or representative of a subject) that are representative of the locus or nucleotide (e.g., having the same identical nucleic acid sequence and/or nucleic acid sequence context, or having a substantially identical nucleic acid sequence and/or nucleic acid context).

*Oligonucleotide primer*: As used herein, the term oligonucleotide primer, or primer, refers to a nucleic acid molecule used, capable of being used, or for use in, generating amplicons from a template nucleic acid molecule. Under transcription-permissive conditions (e.g., in the presence of nucleotides and a DNA polymerase, and at a suitable temperature and pH), an oligonucleotide primer can provide a point of initiation of transcription from a template to which the oligonucleotide primer hybridizes. Typically, an oligonucleotide primer is a single-stranded nucleic acid between 5 and 200 nucleotides in length. Those of skill in the art will appreciate that optimal primer length for generating amplicons from a template nucleic acid molecule can vary with conditions including temperature parameters, primer composition, and transcription or amplification method. A pair of oligonucleotide primers, as used herein, refers to a set of two oligonucleotide primers that are respectively complementary to a first strand and a second strand of a template double-stranded nucleic acid molecule. First and second members of a pair of oligonucleotide primers may be referred to as a "forward" oligonucleotide primer and a "reverse" oligonucleotide primer, respectively, with respect to a template nucleic acid strand, in that the forward oligonucleotide primer is capable of hybridizing with a nucleic acid strand complementary to the template nucleic acid strand, the reverse oligonucleotide primer is capable of hybridizing with the template nucleic acid strand, and the position of the forward oligonucleotide primer with respect to the template nucleic acid strand is 5' of the position of the reverse oligonucleotide primer sequence with respect to the template nucleic acid strand. It will be understood by those of skill in the art that the identification of a first and second oligonucleotide primer as forward and reverse oligonucleotide primers, respectively, is arbitrary inasmuch as these identifiers depend upon whether a given nucleic acid strand or its complement is utilized as a template nucleic acid molecule.

*Overlapping*: The term "overlapping" is used herein in reference to two regions of DNA, each of which contains a sub-sequence that is substantially identical to a sub-sequence of the same length in the other region (e.g., the two regions of DNA have a common sub-sequence). "Substantially identical" means that the two identically-long sub-sequences differ by fewer than a given number of base pairs. In certain instances, e.g., as set forth herein, each sub-sequence has a length of at least 20 base pairs that differ by fewer than 4, 3, 2, or 1 base pairs from each other (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 24 base pairs that differ by fewer than 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 50 base pairs that differ by fewer than 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 100 base pairs that differ by fewer than 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 200 base pairs that differ by fewer than 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 250 base pairs that differ by fewer than 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 300 base pairs that differ by fewer than 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 500 base pairs that differ by fewer than 100, 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 1000 base pairs that differ by fewer than 200, 100, 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, the subsequence of a first region of the two regions of DNA may comprise the entirety of the second region of the two regions of DNA (or vice versa) (e.g., the common sub-sequence may contain the whole of either or both regions). In certain embodiments, where a methylation locus has a sequence that comprises at "least a portion of" a DMR sequence listed herein (e.g., at least 20%, at least 30%, at least 40%, at least 50%, at least 60%, at least 70%, at least 80%, or at least 90% of the DMR sequence), the overlapping portion of the methylation locus has at least 95% similarity, at least 98% similarity, or at least 99% similarity with the overlapping portion of the DMR sequence (e.g., if the overlapping portion is 100bp, the portion of the methylation locus that overlaps with the portion of the DMR differs by no more than 1 bp, no more than 2 bp, or no more than 5 bp). In certain embodiments, where a methylation locus has a sequence that comprises "at least a portion of" a DMR sequence listed herein, this means the methylation locus has a subsequence in common with the DMR sequence that has a consecutive series of bases that covers at least 20%, at least 30%, at least 40%, at least 50%, at least 60%, at least 70%, at least 80%, or at least 90% of the DMR sequence, e.g., wherein the subsequence in common differs by no more than 1 bp, no more than 2 bp, or no more than 5 bp). In certain embodiments, where a methylation locus has a sequence that comprises "at least a portion of" a DMR sequence listed herein, this means the methylation locus contains at least a portion of (e.g., at least 20%, at least 30%, at least 40%, at least 50%, at least 60%, at least 70%, at least 80%, or at least 90% of) the CpG dinucleotides corresponding to the CpG dinucleotides within the DMR sequence.

*Pharmaceutical composition*: As used herein, the term "pharmaceutical composition" refers to a composition in which an active agent is formulated together with one or more pharmaceutically acceptable carriers. In some embodiments, e.g., as set forth herein, the active agent is present in a unit dose amount appropriate for administration to a subject, e.g., in a therapeutic regimen that shows a statistically significant probability of achieving a predetermined therapeutic effect when administered to a relevant population. In some embodiments, e.g., as set forth herein, a pharmaceutical composition can be formulated for administration in a particular form (e.g., in a solid form or a liquid form), and/or can be specifically adapted for, for example: oral administration (for example, as a drenche (aqueous or non-aqueous solutions or suspensions), tablet, capsule, bolus, powder, granule, paste, etc., which can be formulated specifically for example for buccal, sublingual, or systemic absorption); parenteral administration (for example, by subcutaneous, intramuscular, intravenous or epidural injection as, for example, a sterile solution or suspension, or sustained-release formulation, etc.); topical application (for example, as a cream, ointment, patch or spray applied for example to skin, lungs, or oral cavity); intravaginal or intrarectal administration (for example, as a pessary, suppository, cream, or foam); ocular administration; nasal or pulmonary administration, etc.

*Pharmaceutically acceptable:* As used herein, the term "pharmaceutically acceptable," as applied to one or more, or all, component(s) for formulation of a composition as disclosed herein, means that each component must be compatible with the other ingredients of the composition and not deleterious to the recipient thereof.

*Pharmaceutically acceptable carrier:* As used herein, the term "pharmaceutically acceptable carrier" refers to a pharmaceutically-acceptable material, composition, or vehicle, such as a liquid or solid filler, diluent, excipient, or solvent encapsulating material, that facilitates formulation and/or modifies bioavailability of an agent, e.g., a pharmaceutical agent. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

*Polyposis syndromes*: The terms "polyposis" and "polyposis syndrome", as used herein, refer to hereditary conditions that include, but are not limited to, familial adenomatous polyposis (FAP), hereditary nonpolyposis colorectal cancer (HNPCC)/Lynch syndrome, Gardner syndrome, Turcot syndrome, MUTYH polyposis, Peutz-Jeghers syndrome, Cowden disease, familial juvenile polyposis, and hyperplastic polyposis. In certain embodiments, polyposis includes serrated polyposis syndrome. Serrated polyposis is classified by a subject having 5 or more serrated polyps proximal to the sigmoid colon with two or more at least 10 mm in size, having a serrated polyp proximal to the sigmoid colon in the context of a family history of serrated polyposis, and/or having 20 or more serrated polyps throughout the colon.

*Portion* when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

*Prevent or prevention*: The terms "prevent" and "prevention," as used herein in connection with the occurrence of a disease, disorder, or condition, refers to reducing the risk of developing the disease, disorder, or condition; delaying onset of the disease, disorder, or condition; delaying onset of one or more characteristics or symptoms of the disease, disorder, or condition; and/or to reducing the frequency and/or severity of one or more characteristics or symptoms of the disease, disorder, or condition. Prevention can refer to prevention in a particular subject or to a statistical impact on a population of subjects. Prevention can be considered complete when onset of a disease, disorder, or condition has been delayed for a predefined period of time.

*Probe*: As used herein, the terms "probe", "capture probe", or "bait" refer to a single- or double-stranded nucleic acid molecule that is capable of hybridizing with a complementary target and, in certain embodiments, includes a detectable moiety. In certain embodiments, e.g., as set forth herein, a probe is a restriction digest product or is a synthetically produced nucleic acid, e.g., a nucleic acid produced by recombination or amplification. In some instances, e.g., as set forth herein, a probe is a capture probe useful in detection, identification, and/or isolation of a target sequence, such as a gene sequence. In various instances, e.g., as set forth herein, a detectable moiety of probe can be, e.g., an enzyme (e.g., ELISA, as well as enzyme-based histochemical assays), fluorescent moiety, radioactive moiety, or moiety associated with a luminescence signal.

*Prognosis*: As used herein, the term "prognosis" refers to determining the qualitative of quantitative probability of at least one possible future outcome or event. As used herein, a prognosis can be a determination of the likely course of a disease, disorder, or condition such as cancer in a subject, a determination regarding the life expectancy of a subject, or a determination regarding response to therapy, e.g., to a particular therapy.

*Prognostic information*: As used herein, the terms "prognostic information" and "predictive information" are used to refer to any information that may be used to indicate any aspect of the course of a disease or condition either in the absence or presence of treatment. Such information may include, but is not limited to, the average life expectancy of a patient, the likelihood that a patient will survive for a given amount of time (e.g., 6 months, 1 year, 5 years, etc.), the likelihood that a patient will be cured of a disease, the likelihood that a patient's disease will respond to a particular therapy (wherein response may be defined in any of a variety of ways). Prognostic and predictive information are included within the broad category of diagnostic information. Prognostic information can include, without limitation, biomarker status information.

*Promoter:* As used herein, a "promoter" can refer to a DNA regulatory region that directly or indirectly (e.g., through promoter-bound proteins or substances) associates with an RNA polymerase and participates in initiation of transcription of a coding sequence.

*Ratio:* As used herein, the term "ratio" refers to a calculable relationship used to compare amounts of two species that indicates the relative amounts of the species. The species may be markers, such as metabolites and/or lipids (e.g., fatty acids), for example. A ratio may be a direct proportion or inverse proportion (e.g., a first amount divided by a second amount, or the second amount divided by the first amount, respectively). A ratio may be weighted and/or normalized (either the numerator, the denominator, or both). The two amounts may be physical quantities or arbitrary values that correspond to physical quantities. For example, a ratio may be calculated from two intensity amounts (i.e., in arbitrary units) in two species (e.g., markers) measured by a mass spectrometry technique.

*Reference:* As used herein describes a standard or control relative to which a comparison is performed. For example, in some embodiments, e.g., as set forth herein, an agent, subject, animal, individual, population, sample, sequence, or value of interest is compared with a reference or control agent, subject, animal, individual, population, sample, sequence, or value. In some embodiments, e.g., as set forth herein, a reference or characteristic thereof is tested and/or determined substantially simultaneously with the testing or determination of the characteristic in a sample of interest. In some embodiments, e.g., as set forth herein, a reference is a historical reference, optionally embodied in a tangible medium. Typically, as would be understood by those of skill in the art, a reference is determined or characterized under comparable conditions or circumstances to those under assessment, e.g., with regard to a sample. Those skilled in the art will appreciate when sufficient similarities are present to justify reliance on and/or comparison to a particular possible reference or control.

*Risk:* As used herein with respect to a disease, disorder, or condition, the term "risk" refers to the qualitative of quantitative probability (whether expressed as a percentage or otherwise) that a particular individual will develop the disease, disorder, or condition. In some embodiments, e.g., as set forth herein, risk is expressed as a percentage. In some embodiments, e.g., as set forth herein, a risk is a qualitative of quantitative probability that is equal to or greater than 0, 1, 2, 3, 4, 5, 6, 7, 8, 9, 10, 20, 30, 40, 50, 60, 70, 80, 90, or 100%. In some embodiments, e.g., as set forth herein, risk is expressed as a qualitative or quantitative level of risk relative to a reference risk or level or the risk of the same outcome attributed to a reference. In some embodiments, e.g., as set forth herein, relative risk is increased or decreased in comparison to the reference sample by a factor of 1.1, 1.2, 1.3, 1.4, 1.5, 1.6, 1.7,1.8, 1.9, 2, 3, 4, 5, 6, 7, 8, 9, 10, or more.

*Sample:* As used herein, the term "sample" typically refers to an aliquot of material obtained or derived from a source of interest. In some embodiments, e.g., as set forth herein, a source of interest is a biological or environmental source. In some embodiments, e.g., as set forth herein, a sample is a "primary sample" obtained directly from a source of interest. In some embodiments, e.g., as set forth herein, as will be clear from context, the term "sample" refers to a preparation that is obtained by processing of a primary sample (e.g., by removing one or more components of and/or by adding one or more agents to a primary sample). Such a "processed sample" can include, for example cells, nucleic acids, or proteins extracted from a sample or obtained by subjecting a primary sample to techniques such as amplification or reverse transcription of nucleic acids, isolation and/or purification of certain components, *etc.*

In certain instances, e.g., as set forth herein, a processed sample can be a DNA sample that has been amplified (e.g., pre-amplified). Thus, in various instances, e.g., as set forth herein, an identified sample can refer to a primary form of the sample or to a processed form of the sample. In some instances, e.g., as set forth herein, a sample that is enzyme-digested DNA can refer to primary enzyme-digested DNA (the immediate product of enzyme digestion) or a further processed sample such as enzyme-digested DNA that has been subject to an amplification step (e.g., an intermediate amplification step, e.g., pre-amplification) and/or to a filtering step, purification step, or step that modifies the sample to facilitate a further step, e.g., in a process of determining methylation status (e.g., methylation status of a primary sample of DNA and/or of DNA as it existed in its original source context).

*Screening*: As used herein, the term "screening" refers to any method, technique, process, or undertaking intended to generate diagnostic information and/or prognostic information. Accordingly, those of skill in the art will appreciate that the term screening encompasses method, technique, process, or undertaking that determines whether an individual has, is likely to have or develop, or is at risk of having or developing a disease, disorder, or condition, e.g., pancreatic cancer, colorectal cancer, advanced adenoma.

*Specificity*: As used herein, the "specificity" of a biomarker refers to the percentage of samples that are characterized by absence of the event or state of interest for which measurement of the biomarker accurately indicates absence of the event or state of interest (true negative rate). In various embodiments, e.g., as set forth herein, characterization of the negative samples is independent of the biomarker, and can be achieved by any relevant measure, e.g., any relevant measure known to those of skill in the art. Thus, specificity reflects the probability that the biomarker would detect the absence of the event or state of interest when measured in a sample not characterized that event or state of interest. In particular embodiments in which the event or state of interest is pancreatic cancer, e.g., as set forth herein, specificity refers to the probability that a biomarker would detect the absence of pancreatic cancer in a subject lacking pancreatic cancer. Lack of pancreatic cancer can be determined, e.g., by histology.

*Sensitivity*: As used herein, the "sensitivity" of a biomarker refers to the percentage of samples that are characterized by the presence of the event or state of interest for which measurement of the biomarker accurately indicates presence of the event or state of interest (true positive rate). In various embodiments, e.g., as set forth herein, characterization of the positive samples is independent of the biomarker, and can be achieved by any relevant measure, e.g., any relevant measure known to those of skill in the art. Thus, sensitivity reflects the probability that a biomarker would detect the presence of the event or state of interest when measured in a sample characterized by presence of that event or state of interest. In particular embodiments in which the event or state of interest is pancreatic cancer, e.g., as set forth herein, sensitivity refers to the probability that a biomarker would detect the presence of pancreatic cancer in a subject that has pancreatic cancer. Presence of pancreatic cancer can be determined, e.g., by histology.

*Single Nucleotide Polymorphism (SNP)*: As used herein, the term "single nucleotide polymorphism" or "SNP" refers to a particular base position in the genome where alternative bases are known to distinguish one allele from another. In some embodiments, one or a few SNPs and/or CNPs is/are sufficient to distinguish complex genetic variants from one another so that, for analytical purposes, one or a set of SNPs and/or CNPs may be considered to be characteristic of a particular variant, trait, cell type, individual, species, etc, or set thereof. In some embodiments, one or a set of SNPs and/or CNPs may be considered to define a particular variant, trait, cell type, individual, species, etc, or set thereof.

*Solid Tumor*: As used herein, the term "solid tumor" refers to an abnormal mass of tissue including cancer cells. In various embodiments, e.g., as set forth herein, a solid tumor is or includes an abnormal mass of tissue that does not contain cysts or liquid areas. In some embodiments, e.g., as set forth herein, a solid tumor can be benign; in some embodiments, a solid tumor can be malignant. Examples of solid tumors include carcinomas, lymphomas, and sarcomas. In some embodiments, e.g., as set forth herein, solid tumors can be or include adrenal, bile duct, bladder, bone, brain, breast, cervix, colon, endometrium, esophagum, eye, gall bladder, gastrointestinal tract, kidney, larynx, liver, lung, nasal cavity, nasopharynx, oral cavity, ovary, penis, pituitary, prostate, retina, salivary gland, skin, small intestine, stomach, testis, thymus, thyroid, uterine, vaginal, and/or vulval tumors.

*Stage of cancer:* As used herein, the term "stage of cancer" refers to a qualitative or quantitative assessment of the level of advancement of a cancer. In some embodiments, e.g., as set forth herein, criteria used to determine the stage of a cancer can include, but are not limited to, one or more of where the cancer is located in a body, tumor size, whether the cancer has spread to lymph nodes, whether the cancer has spread to one or more different parts of the body, etc. In some embodiments, e.g., as set forth herein, cancer can be staged using the so-called TNM System, according to which T refers to the size and extent of the main tumor, usually called the primary tumor; N refers to the number of nearby lymph nodes that have cancer; and M refers to whether the cancer has metastasized. In some embodiments, e.g., as set forth herein, a cancer can be referred to as Stage 0 (abnormal cells are present but have not spread to nearby tissue, also called carcinoma *in situ,* or CIS; CIS is not cancer, but it can become cancer), Stage I-III (cancer is present; the higher the number, the larger the tumor and the more it has spread into nearby tissues), or Stage IV (the cancer has spread to distant parts of the body). In some embodiments, e.g., as set forth herein, a cancer can be assigned to a stage selected from the group consisting of: *in situ* (abnormal cells are present but have not spread to nearby tissue); localized (cancer is limited to the place where it started, with no sign that it has spread); regional (cancer has spread to nearby lymph nodes, tissues, or organs): distant (cancer has spread to distant parts of the body); and unknown (there is not enough information to identify cancer stage).

*Stratification:* Herein, "stratification" refers to any analytical process in which the patients will be classified in separate groups. The groups can share some similar features or characteristics that make them unique as a group. Stratification can be used for any of the study features that will help for the purpose of PaCa diagnostic prediction, early detection, monitoring, treatment guidance and survival prognosis.

*Survival:* Herein, "survival" refers to the time duration the patient has lived since the start of a disease (e.g., cancer) or start of a treatment. This is a term with which can be described how effective a novel approach (including, prognosis, screening, diagnosis, treatment, and monitoring, etc.,..) is in face of disease progression.

_Susceptible to_: An individual who is "susceptible to" a disease, disorder, or condition is at risk for developing the disease, disorder, or condition. In some embodiments, e.g., as set forth herein, an individual who is susceptible to a disease, disorder, or condition does not display any symptoms of the disease, disorder, or condition. In some embodiments, e.g., as set forth herein, an individual who is susceptible to a disease, disorder, or condition has not been diagnosed with the disease, disorder, and/or condition. In some embodiments, e.g., as set forth herein, an individual who is susceptible to a disease, disorder, or condition is an individual who has been exposed to conditions associated with, or presents a biomarker status (e.g., a methylation status) associated with, development of the disease, disorder, or condition. In some embodiments, e.g., as set forth herein, a risk of developing a disease, disorder, and/or condition is a population-based risk (e.g., family members of individuals suffering from the disease, disorder, or condition).

_Subject:_ As used herein, the term "subject" refers to an organism, typically a mammal (e.g., a human). In some embodiments, e.g., as set forth herein, a subject is suffering from a disease, disorder or condition. In some embodiments, e.g., as set forth herein, a subject is susceptible to a disease, disorder, or condition. In some embodiments, e.g., as set forth herein, a subject displays one or more symptoms or characteristics of a disease, disorder or condition. In some embodiments, e.g., as set forth herein, a subject is not suffering from a disease, disorder or condition. In some embodiments, e.g., as set forth herein, a subject does not display any symptom or characteristic of a disease, disorder, or condition. In some embodiments, e.g., as set forth herein, a subject is someone with one or more features characteristic of susceptibility to or risk of a disease, disorder, or condition. In some embodiments, e.g., as set forth herein, a subject is a patient. In some embodiments, e.g., as set forth herein, a subject is an individual to whom diagnosis has been performed and/or to whom therapy has been administered. In some instances, e.g., as set forth herein, a human subject can be interchangeably referred to as an "individual."

_Therapeutic agent:_ As used herein, the term "therapeutic agent" refers to any agent that elicits a desired pharmacological effect when administered to a subject. In some embodiments, e.g., as set forth herein, an agent is considered to be a therapeutic agent if it demonstrates a statistically significant effect across an appropriate population. In some embodiments, e.g., as set forth herein, the appropriate population can be a population of model organisms or a human population. In some embodiments, e.g., as set forth herein, an appropriate population can be defined by various criteria, such as a certain age group, gender, genetic background, preexisting clinical conditions, etc. In some embodiments, e.g., as set forth herein, a therapeutic agent is a substance that can be used for treatment of a disease, disorder, or condition. In some embodiments, e.g., as set forth herein, a therapeutic agent is an agent that has been or is required to be approved by a government agency before it can be marketed for administration to humans. In some embodiments, e.g., as set forth herein, a therapeutic agent is an agent for which a medical prescription is required for administration to humans.

_Therapeutically effective amount:_ As used herein, the term "therapeutically effective amount" refers to an amount that produces a desired effect for which it is administered. In some embodiments, e.g., as set forth herein, the term refers to an amount that is sufficient, when administered to a population suffering from or susceptible to a disease, disorder, or condition, in accordance with a therapeutic dosing regimen, to treat the disease, disorder, or condition. Those of ordinary skill in the art will appreciate that the term therapeutically effective amount does not in fact require successful treatment be achieved in a particular individual. Rather, a therapeutically effective amount can be an amount that provides a particular desired pharmacological response in a significant number of subjects when administered to individuals in need of such treatment. In some embodiments, e.g., as set forth herein, reference to a therapeutically effective amount can be a reference to an amount as measured in one or more specific tissues (e.g., a tissue affected by the disease, disorder or condition) or fluids (e.g., blood, saliva, serum, sweat, tears, urine, etc.). Those of ordinary skill in the art will appreciate that, in some embodiments, a therapeutically effective amount of a particular agent can be formulated and/or administered in a single dose. In some embodiments, e.g., as set forth herein, a therapeutically effective agent can be formulated and/or administered in a plurality of doses, for example, as part of a multi-dose dosing regimen.

_Treatment_: As used herein, the term "treatment" (also "treat" or "treating") refers to administration of a therapy that partially or completely alleviates, ameliorates, relieves, inhibits, delays onset of, reduces severity of, and/or reduces incidence of one or more symptoms, features, and/or causes of a particular disease, disorder, or condition, or is administered for the purpose of achieving any such result. In some embodiments, e.g., as set forth herein, such treatment can be of a subject who does not exhibit signs of the relevant disease, disorder, or condition and/or of a subject who exhibits only early signs of the disease, disorder, or condition. Alternatively or additionally, such treatment can be of a subject who exhibits one or more established signs of the relevant disease, disorder and/or condition. In some embodiments, e.g., as set forth herein, treatment can be of a subject who has been diagnosed as suffering from the relevant disease, disorder, and/or condition. In some embodiments, e.g., as set forth herein, treatment can be of a subject known to have one or more susceptibility factors that are statistically correlated with increased risk of development of the relevant disease, disorder, or condition. In various examples, treatment is of a cancer.

_Upstream_: As used herein, the term "upstream" means a first DNA region is closer, relative to a second DNA region, to the N-terminus of a nucleic acid that includes the first DNA region and the second DNA region.

*Unit dose*: As used herein, the term "unit dose" refers to an amount administered as a single dose and/or in a physically discrete unit of a pharmaceutical composition. In many embodiments, e.g., as set forth herein, a unit dose contains a predetermined quantity of an active agent. In some embodiments, e.g., as set forth herein, a unit dose contains an entire single dose of the agent. In some embodiments, e.g., as set forth herein, more than one unit dose is administered to achieve a total single dose. In some embodiments, e.g., as set forth herein, administration of multiple unit doses is required, or expected to be required, in order to achieve an intended effect. A unit dose can be, for example, a volume of liquid (e.g., an acceptable carrier) containing a predetermined quantity of one or more therapeutic moieties, a predetermined amount of one or more therapeutic moieties in solid form, a sustained release formulation or drug delivery device containing a predetermined amount of one or more therapeutic moieties, etc. It will be appreciated that a unit dose can be present in a formulation that includes any of a variety of components in addition to the therapeutic agent(s). For example, acceptable carriers (e.g., pharmaceutically acceptable carriers), diluents, stabilizers, buffers, preservatives, etc., can be included. It will be appreciated by those skilled in the art, in many embodiments, e.g., as set forth herein, a total appropriate daily dosage of a particular therapeutic agent can comprise a portion, or a plurality, of unit doses, and can be decided, for example, by a medical practitioner within the scope of sound medical judgment. In some embodiments, e.g., as set forth herein, the specific effective dose level for any particular subject or organism can depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts.

*Unmethylated*: As used herein, the terms "unmethylated" and "non-methylated" are used interchangeably and mean that an identified DNA region includes no methylated nucleotides.

*Variant:* As used herein, the term "variant" refers to an entity that shows significant structural identity with a reference entity but differs structurally from the reference entity in the presence, absence, or level of one or more chemical moieties as compared with the reference entity. In some embodiments, e.g., as set forth herein, a variant also differs functionally from its reference entity. In general, whether a particular entity is properly considered to be a "variant" of a reference entity is based on its degree of structural identity with the reference entity. A variant can be a molecule comparable, but not identical to, a reference. For example, a variant nucleic acid can differ from a reference nucleic acid at one or more differences in nucleotide sequence. In some embodiments, e.g., as set forth herein, a variant nucleic acid shows an overall sequence identity with a reference nucleic acid that is at least 85%, 86%, 87%, 88%, 89%, 90%, 91%, 92%, 93%, 94%, 95%, 96%, 97%, or 99%. In many embodiments, e.g., as set forth herein, a nucleic acid of interest is considered to be a "variant" of a reference nucleic acid if the nucleic acid of interest has a sequence that is identical to that of the reference but for a small number of sequence alterations at particular positions. In some embodiments, e.g., as set forth herein, a variant has 10, 9, 8, 7, 6, 5, 4, 3, 2, or 1 substituted residues as compared with a reference. In some embodiments, e.g., as set forth herein, a variant has not more than 5, 4, 3, 2, or 1 residue additions, substitutions, or deletions as compared with the reference. In various embodiments, e.g., as set forth herein, the number of additions, substitutions, or deletions is fewer than about 25, about 20, about 19, about 18, about 17, about 16, about 15, about 14, about 13, about 10, about 9, about 8, about 7, about 6, and commonly are fewer than about 5, about 4, about 3, or about 2 residues.

**BRIEF DESCRIPTION OF THE DRAWINGS**

The foregoing and other objects, aspects, features, and advantages of the present disclosure will become more apparent and better understood by referring to the following description taken in conjunction with the accompanying drawings, in which:

FIG. 1 is a schematic diagram illustrating DNA methylation in normal cells vs. cancer cells.
FIG. 2 is a block flow diagram of a workflow for markers discovery, according to an illustrative embodiment.
FIG. 3 is a heat map demonstrating hierarchical clustering of markers discovered from PAAD-TCGA, according to an illustrative embodiment.
FIG. 4 is a schematic diagram demonstrating Gene Ontologies (GOs) biological process and molecular function analysis, according to an illustrative embodiment.
FIG. 5 is a schematic diagram demonstrating pathway analysis, according to an illustrative embodiment.
FIG. 6 are graphs demonstrating a Kaplan-Meier analysis on hierarchical clustered divided patient groups from TCGA-PAAD, according to an illustrative embodiment.

**DETAILED DESCRIPTION**

It is contemplated that systems, architectures, devices, methods, and processes of the claimed invention encompass variations and adaptations developed using information from the embodiments described herein. Adaptation and/or modification of the systems, architectures, devices, methods, and processes described herein may be performed, as contemplated by this description.

Throughout the description, where articles, devices, systems, and architectures are described as having, including, or comprising specific components, or where processes and methods are described as having, including, or comprising specific steps, it is contemplated that, additionally, there are articles, devices, systems, and architectures of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim.

Documents are incorporated herein by reference as noted. Where there is any discrepancy in the meaning of a particular term, the meaning provided in the Definition section above is controlling.

Headers are provided for the convenience of the reader - the presence and/or placement of a header is not intended to limit the scope of the subject matter described herein.

Steps of the methods described herein may be performed with the assistance of a computing device. For example, certain steps may be performed by a processor of a computing device. Method steps may be performed using a processor of a computing device and a memory having instructions stored thereon, wherein the instructions, when executed by the processor, cause the processor to perform the steps.

Methylation Assays

Methylation state of the specified markers can be assessed with different technological approaches and as such, is not restricted to any specific method by which a gene's methylation state is measured. For example, methylation state can be measured by a genome sequencing method where whole genome is scanned on base-pair resolution. Another method can involve analyzing changes in methylation patterns with a PCR-based process that involves digestion of DNA with methylation-sensitive restriction enzymes prior to PCR amplification.

In the context of MSRE-qPCR, the amount of total DNA is determined by directly measuring the portion of extracted DNA with real-time or digital PCR in its native form. Then, part of the DNA is digested with restriction enzymes that degrades unmethylated DNA, leaving only methylated strands intact. Resulting methylated sequences are then determined using once again either a real-time PCR or digital PCR.

Real-time PCR-based methods comprise generating a standard curve for the unmethylated target by using external standards. The standard curve is constructed from at least two points and relates the real-time Ct value for undigested and digested DNA to known quantitative standards. Next, the test sample Ct values are determined for the digested and undigested populations and the genomic equivalents of DNA are calculated from the standard curves produced. Ct values of undigested and digested DNA are evaluated to establish markers that were truly digested and thus producing a Ct values of 45 and markers that failed to be amplified from the samples and can be considered as failed values. Filtered and corrected values from digested DNA can then be compared directly between different condition groups to establish the relative differences in methylation levels between the groups. Additionally, delta-difference between the Ct values of undigested and digested DNA can be used for the same purpose.

Digital PCR based methods involve distributing a reaction across a 96- or 384-well plate or higher in a microfluidic device, such that the mean initial template DNA concentration is less than one molecule per reaction compartment. Amplification of methylated DNA molecules occurs in a small minority of PCR wells, and therefore represents a digital readout of the original number of template molecules in each sample.

In addition, other techniques that utilize bisulfite treatment of DNA as a starting point for methylation analysis can be used. These include methylation-specific PCR (MSP) (Herman et al. (1992) Proc. Natl. Acad. 20 Sci. USA 93: 9821-9826), Methylation Specific Nuclease assisted Minor-allele Enrichment (MS-NaME)- PCR (Liu Y et al. Nucleic Acids Res. 2017 Apr 7;45(6):e39.), Methylation-Sensitive High-Resolution Melting (MS-HRM) PCR (Hussmann D, Hansen LL. Methods Mol Biol. 2018;1708:551-571.), the texts of which are incorporated herein by reference in their entireties.

For evaluating a methylation state, the methylation state is often expressed as the fraction or percentage of individual strands of DNA that is methylated at a particular site (e.g., at a single nucleotide, at a particular region or locus, at a longer sequence of interest, e.g., up to a ~50-bp, 100-bp, 150-bp, 200-bp,500-bp, 1000-bp subsequence of a DNA or longer) relative to the total population of DNA in the sample comprising that particular site.

Neoplasia of a biological sample can also be indicated when a methylation ratio of one or more DNA methylation markers relative to a level of bisulfite-treated DNA copy number of a reference gene is different, wherein the one or more DNA methylation markers comprises a base in a differentially methylated region (DMR). The methylation ratio includes the ratio of the methylation level of the DNA methylation marker and the level of a region in a reference gene determined by the same means used for the determination of the methylation level of the biomarker. Usually, the methylation ratio is represented by the ratio of the methylation level of the DNA methylation marker and the level of a region in a reference gene determined by the same means used for the determination of the methylation level of the DNA methylation marker.

The methylation ratio can be the ratio of the methylation level of a DNA methylation marker and the level of a region of a reference gene, both of which are quantitatively measured using real-time polymerase chain reaction (PCR) or by droplet digital PCR (ddPCR) method. For example, the methylation level of a DNA methylation marker from a sample of a subject can be quantitatively measured using a pair of primers and an oligonucleotide probe, where one primer, both primers, the oligonucleotide probe, or both primers and the oligonucleotide probe are capable of distinguishing between and selectively amplifying of methylated and unmethylated nucleic acid, e.g., after the nucleic acid has been modified by a modifying agent, e.g., digesting the unmethylated DNA with methylation specific enzymes or using bisulfite for converting unmethylated cytosine to a converted nucleic acid.

Biomarker discovery

An Initial Discovery study was undertaken and included data from The Cancer Genome Atlas (TCGA) Research Network (http://cancergenome.nih.gov/) for PaCa (TCGA-PAAD) and normal tissue.

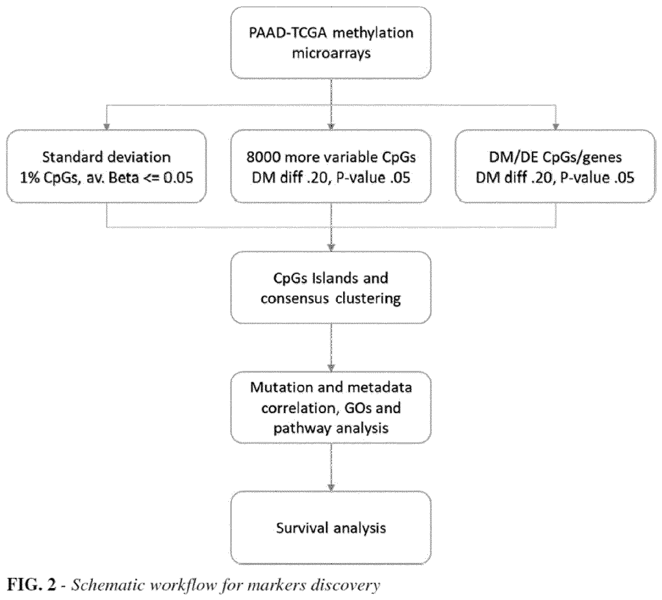

For significant marker selection, our analysis consisted of three steps (see FIG. 2). First, the 8000 more variable probes were obtained from the TCGA-PAAD methylation datasets (Illumina 450k methylation arrays) and were subjected to an Enhancer Linking by Methylation/Expression Relationships algorithm to relate the methylation levels to gene expression.

The comparison was performed on supervised mode, assigning manually two groups to be contrasted as healthy tissue and tumor tissue. Fisher's exact test and Benjamini-Hochberg multiple hypothesis correction was used to compare frequency of each motif flanking the positive CpG probes to a background defined by all distal probes on the array. Those CpGs that passed the minimum threshold (significant methylation difference 0.2, p value 0.05), were considered as markers.

Second, we obtained all the distal probes from TCGA-PAAD methylation dataset and performed the Enhancer Linking by Methylation/Expression Relationships analysis, where, after a differential methylation analysis (DMA), both hyper and hypomethylated probes are related to gene expression to cross-validate DMA analysis. CpGs that passed the minimum threshold (significant methylation difference 0.2, p value 0.05) were considered as markers.

Third, the 1% of CpGs from tumoral samples with highest standard deviation and an average beta value in normal samples less than 0.05 were considered significant and used for the next step.

All the markers obtained from the preceding steps were summed together, eliminating the repeated probes. Afterward, the CpGs features were filtered, picking only the CpGs occurring on islands and underwent to consensus clustering in order to define the optimal cluster number. An enrichment analysis (hypergeometric distribution) was performed to assess the significance of different metadata information inside each cluster. To investigate whether the selected markers represent biological processes relevant to the carcinogenic transformation, we performed a Gene ontology and biological pathway analysis. Finally, in order to assess the survival function, each marker set form each cancer type was evaluated using a Kaplan-Meier analysis.

Results

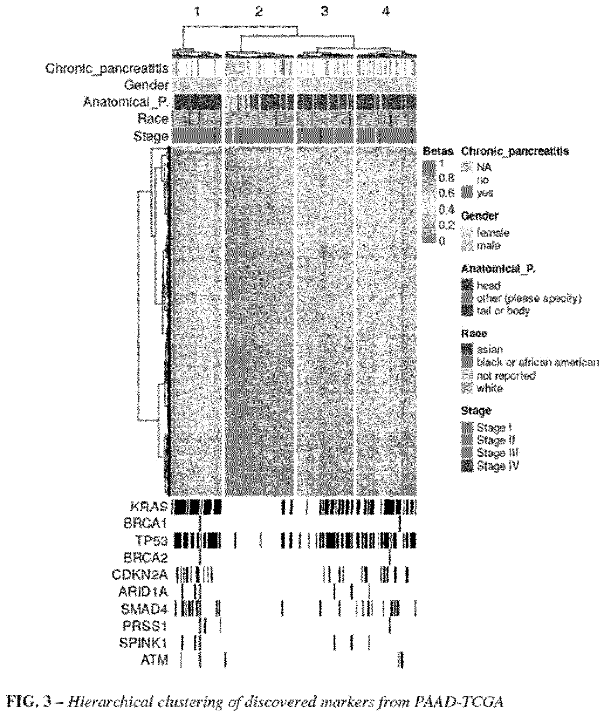

Epigenetic classes were defined by patterns in the methylation of sub-genomic components. Consensus clustering of PAAD dataset identified four clusters; a highly methylated cluster with progressive hypomethylation on subsequent clusters (see FIG. 3).

PAAD presents enrichment on the highly methylated cluster on driver mutations as KRAS and TP53 and SMAD4 and CDKN2A in the highly methylated and intermediate clusters.

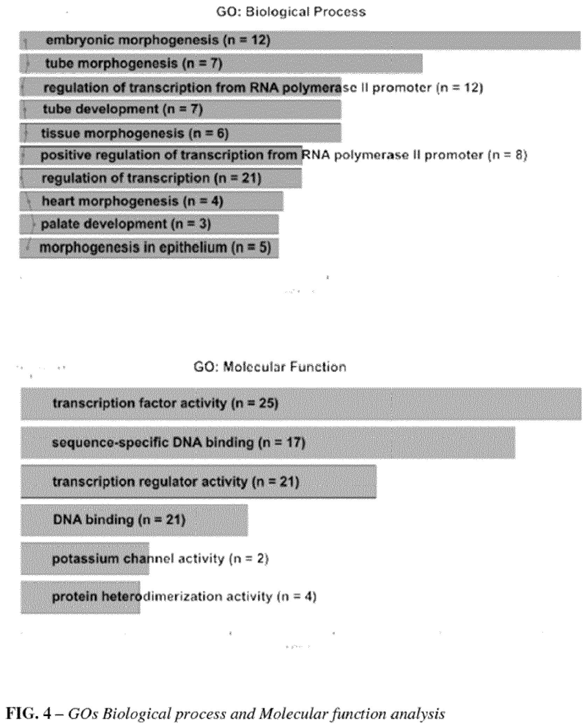

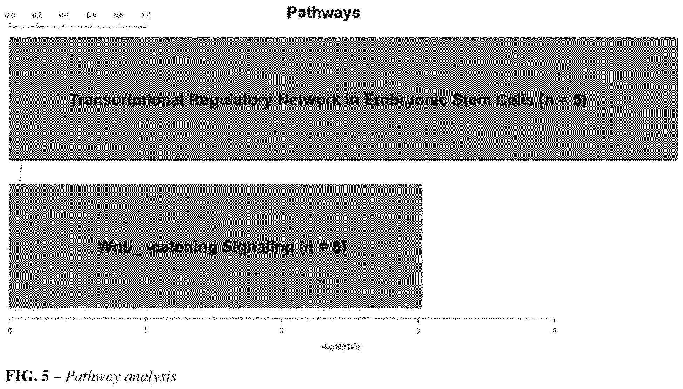

A goal of the study is to identify methylation patterns that potentially are linked to clonal evolution in a particular tumor type. In order to archive this, we have carried out a Gene Ontology (GOs) and pathway analysis to capture the biological processes related to regulatory networks, morphogenesis, development and cell differentiation. PAAD (see FIG. 4) showed GOs related to morphogenesis, development and regulation of transcription being over-represented. Biological pathways (see FIG. 5) only showed two members, Transcriptional regulatory network in Embryonic Stem Cells and Wnt/catenin signaling, suggesting the molecular processes that had originated each group.

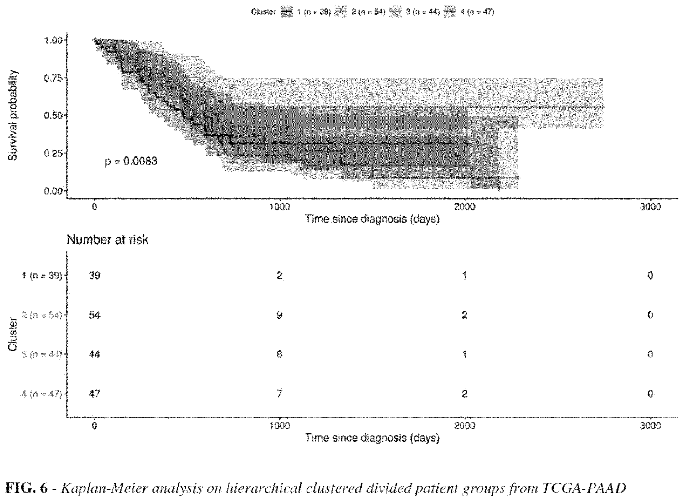

Kaplan-Meier analysis PAAD (see FIG. 6) with a p = 0.0083 demonstrates that patients on the hypomethylated cluster (2) had a better survival chance that the others, patients at hypermethylated cluster died quicker but at similar rate that those on clusters 3 and 4.

Methods for assaying methylation state of selected markers

Target nucleic acid can be isolated from a sample through, different approaches, for example, via a direct gene capture by for example isolating target nucleic acid from a sample through, for example, removal of assay inhibiting agents to produce a clarified sample, capture of a target nucleic acid (if present) from the clarified sample with a capture reagent to form a capture complex, isolating the capture complex from the clarified sample, recovering the target nucleic acid (if present) from the capture complex in a nucleic acid solution.

Fragments of the isolated DNA are amplified using sets of primer oligonucleotides and an amplification enzyme. The amplification of several DNA segments can be carried out simultaneously in one and the same reaction vessel. Typically, the amplification is carried out using a polymerase chain reaction (PCR). Amplicons are typically 100 to 2000 base pairs in length.

DNA amplification can then be followed by further treatment of the DNA with methylation-specific enzymes and measuring the methylation status with qPCR or ddPCR. MSRE-qPCR is a method for analyzing a nucleic acid for the presence of 5-methylcytosine based upon methylation specific restriction enzyme method described by Beikircher, et al. Methods Mol Biol. 2018;1708:407-424 or variations. Restriction enzymes have been used for years for investigating DNA methylation patterns. Generally, methylation-sensitive restriction enzymes (MSREs), which contain CpG motifs in their recognition site are used. Since the activity of these enzymes is blocked by 5-methylcytosine, then only unmethylated sites are digested while methylated regions remain unaffected. Thus, upon successful digestion followed by PCR amplification, only methylated DNA should result in a detectable PCR product.

Design of MSRE-qPCR assays is usually less complicated than the design of bisulfite-based assays, as "native" DNA is targeted with only conditions that primers must cover target regions presenting at least one MSRE-cut-site, but more cut-sites give a better outcome. CpG-rich regions, which are mostly also candidate regions for methylation differences, are good targets for MSRE-qPCR assay design, as they typically contain a large number of suitable MSRE cut-sites. The use of more than one MSRE, in particular restriction enzymes AciI, Hin6I, HpyCH4IV, and Hpall usually provide a very good coverage of CpG-rich sequences. The availability of sample is often a limiting factor, especially in the context of analyzing cell-free DNA, but this can be overcome by using preamplification upon MSRE digestion. Methylation-specific restriction enzyme approach can also be combined with digital PCR.

Methylation markers may be also detected by use of methylation-specific primer oligonucleotides. This technique (MSP) has been described in U.S. Pat. No. 6,265,171 to Herman, the text of which is incorporated herein by reference in its entirety. The use of methylation status specific primers for the amplification of bisulfite treated DNA allows the differentiation between methylated and unmethylated nucleic acids. MSP primer pairs contain at least one primer that hybridizes to a bisulfite treated CpG dinucleotide. Therefore, the sequence of said primers comprises at least one CpG dinucleotide. MSP primers specific for non-methylated DNA contain a "T" at the position of the C position in the CpG.

Another method for analyzing a nucleic acid for the presence of 5-methylcytosine is based upon the bisulfite method described by Frommer, et al. for the detection of 5-methylcytosines in DNA (Frommer et al. Proc Natl Acad Sci U S A. 1992 Mar 1 ;89(5):1827-31) or variations thereafter.

The bisulfite method of mapping 5-methylcytosines is based on the observation that cytosine, but not 5-methylcytosine, reacts with hydrogen sulfite ion (also known as bisulfite). Therefore, DNA that has been treated with bisulfite retains only methylated cytosines. Various methylation assay procedures can be used in conjunction with bisulfite treatment according to the present technology. These assays allow for determination of the methylation state of one or a many of CpG dinucleotides (e.g., CpG islands) within a nucleic acid sequence. Such assays involve, among other techniques, sequencing of bisulfite-treated nucleic acid, PCR (for sequence-specific amplification), Methylation-Specific PCR, Methylation Specific Nuclease-assisted Minor-allele Enrichment PCR, Methylation-Specific Restriction Enzyme qPCR, Methylation-Sensitive High-Resolution Melting.

Targeted sequencing-based protocols entail PCR amplification of target regions of interest from bisulfite-converted genomic DNA, followed by DNA sequencing library preparation using techniques such as standard Illumina protocols or transposase-based Nextera XT technology. Next-generation sequencing (NGS) techniques offer single base resolution reading of CpG methylation levels.

Additionally, assays such as "MethyLight™" (a fluorescence-based real-time PCR technique) (Campan M et al. Methods Mol Biol. 2018;1708:497-513.) can be used for evaluation of the methylation status. MethyLight is a quantitative, fluorescence-based, real-time PCR method to sensitively detect and quantify DNA methylation of candidate regions of the genome. MethyLight is uniquely suited for detecting low-frequency methylated DNA regions against a high background of unmethylated DNA, as it combines methylation-specific priming with methylation-specific fluorescent probing (Campan M et al. Methods Mol Biol. 2018;1708:497-513.). Additionally, MethyLight can be combined with Digital PCR, for the highly sensitive detection of individual methylated molecules, with use in disease detection and screening (Campan M et al. Methods Mol Biol. 2018;1708:497-513.).

MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996). MSP allows for highly sensitive detection (detection level of 0.1% of the alleles, with full specificity) of locus-specific DNA methylation, using PCR amplification of bisulfite-converted DNA. The bisulfite-modified DNA is PCR amplified using specific primers sets, one that binds specifically to the methylated sequence and other that only binds to the unmethylated sequence. MSP results can then be obtained using gel electrophoresis, not requiring further restriction or sequencing analysis.

The Quantitative Multiplex Methylation-Specific PCR (QM-MSP) is another method for sensitive quantification of DNA methylation by using methylation specific primers (Fackler MJ, Sukumar S. Methods Mol Biol. 2018;1708:473-496). QM-MSP is a two-step PCR approach, where in the first step, one pair of gene-specific primers (forward and reverse) amplifies the methylated and unmethylated copies of the same gene simultaneously and in multiplex, in one PCR reaction. This methylation-independent amplification step produces amplicons of up to $10^9$ copies per $\mu$L after 36 cycles of PCR. In the second step, the amplicons of the first reaction are quantified with a standard curve using real-time PCR and two independent fluorophores to detect methylated/unmethylated DNA of each gene in the same well (e.g., 6FAM and VIC). One methylated copy is detectable in 100,000 reference gene copies.

Methylation-Sensitive High-Resolution Melting (MS-HRM) (Hussmann D, Hansen LL. Methods Mol Biol. 2018;1708:551-571.). Methylation-Sensitive High-Resolution Melting (MS-HRM) is an in-tube, PCR-based method to detect methylation levels at specific loci of interest. A unique primer design facilitates a high sensitivity of the assays enabling detection of down to 0.1-1% methylated alleles in an unmethylated background.

Primers for MS-HRM assays are designed to be complementary to the methylated allele, and a specific annealing temperature enables these primers to anneal both to the methylated and the unmethylated alleles thereby increasing the sensitivity of the assays. Bisulfite treatment of the DNA prior to performing MS-HRM ensures a different base composition between methylated and unmethylated DNA, which is used to separate the resulting amplicons by high resolution melting.

MS-NaME (Methylation Specific Nuclease-assisted Minor-allele Enrichment) reaction (Liu Y et al. Nucleic Acids Res. 2017 Apr 7;45(6):e39.) can be used alone or in combination with one or more of these methods. Minor-allele Enrichment (MS-NaME) employs a double-strand-specific DNA nuclease (DSN) to remove excess DNA with normal methylation patterns. The technique utilizes oligonucleotideprobes that direct DSN activity to multiple targets in bisulfite-treated DNA, simultaneously. Oligonucleotide probes targeting unmethylated sequences generate local double stranded regions resulting to digestion of unmethylated targets and leaving methylated targets intact; and vice versa. Subsequent amplification of the targeted regions results in enrichment of the targeted methylated or unmethylated minority-epigenetic-alleles.

Ms-SNuPE™ (Methylation-sensitive Single Nucleotide Primer Extension) reactions (Gonzalgo ML & Liang G, Nat Protoc. 2007;2(8):1931-6.), Strand-specific PCR can be performed to generate a DNA template for quantitative methylation analysis using Ms-SNuPE. SNuPE is then performed with oligonucleotide(s) designed to hybridize immediately upstream of the CpG site(s) being interrogated. Reaction products are electrophoresed on polyacrylamide gels for visualization and quantitation by phosphor-image analysis.

The fragments obtained via amplification can also carry directly or indirectly detectable labels such as fluorescent labels, radionuclides, or detachable molecule fragments having a typical mass that can be detected in a mass spectrometer. The detection may be carried out and visualized by means of, e.g., matrix assisted laser desorption/ionization mass spectrometry (MALDI) or using electron spray mass spectrometry (ESI).

Pancreatic Cancer

In certain embodiments, methods and compositions of the present disclosure are useful for screening for pancreatic cancer.

Pancreatic cancers include pancreatic cancers at any of the various possible stages known in the art, including, e.g., Stage 0, Stage I, Stage II, Stage III, and Stage IV pancreatic cancers. Pancreatic cancers include all stages of the Tumor/Node/Metastasis (TNM) staging system. With respect to pancreatic cancer, T can refer to whether the tumor is confined to the top layers of pancreatic duct cells or has not invaded deeper tissues; N can refer to whether the tumor has spread to lymph nodes, and if so how many lymph nodes and where they are located; and M can refer to whether the cancer has spread to other parts of the body, and if so which parts and to what extent. Particular stages of T, N, and M are known in the art. T stages can include TX, T0, Tis, T1, T2, T3, T4; N stages can include NX, N0, N1, N2; M stages can include M0, M1.

In certain instances, the present disclosure includes screening of early stage pancreatic cancer. Early stage pancreatic cancers can include, e.g., pancreatic cancers localized within a subject, e.g., in that they have not yet spread to lymph nodes of the subject, e.g., lymph nodes near to the cancer (stage N0), and have not spread to distant sites (stage M0). Early stage cancers include pancreatic cancers corresponding to, e.g., Stages 0 to II.

Methods and compositions of the present disclosure are useful for stratifying of pancreatic cancer in all of its forms and stages, including without limitation those named herein or otherwise known in the art, as well as all subsets thereof. Accordingly, the person of skill in art will appreciate that all references to pancreatic cancer provided here include, without limitation, pancreatic cancer in all of its forms and stages, including without limitation those named herein or otherwise known in the art, as well as all subsets thereof.

Subjects and Samples

A sample analyzed using methods and compositions provided herein can be any biological sample and/or any sample including nucleic acids. In various particular embodiments, a sample analyzed using methods and compositions provided herein can be a sample from a mammal. In various particular embodiments, a sample analyzed using methods and compositions provided herein can be a sample from a human subject. In various particular embodiments, a sample analyzed using methods and compositions provided herein can be a sample form a mouse, rat, pig, horse, chicken, or cow.

In various instances, a human subject is a subject diagnosed or seeking diagnosis as having, diagnosed as or seeking diagnosis as at risk of having, and/or diagnosed as or seeking diagnosis as at immediate risk of having, pancreatic cancer. In various instances, a human subject is a subjected identified as a subject in need of screening for pancreatic cancer. In certain instances, a human subject is a subject identified as in need of pancreatic cancer screening by a medical practitioner. In various instances, a human subject is identified as in need of pancreatic cancer screening due to age, e.g., due to an age equal to or greater than 40 years, e.g., an age equal to or greater than 49, 45, 50, 55, 60, 65, 70, 75, 80, 85, or 90 years, though in some instances a subject 18 years old or older may be identified as at risk and/or in need of screening for pancreatic cancer. In various instances, a human subject is identified as being high risk and/or in need of screening for pancreatic cancer based on, without limitation, familial history, prior diagnoses, and/or an evaluation by a medical practitioner. In various instances, a human subject is a subject not diagnosed as having, not at risk of having, not at immediate risk of having, not diagnosed as having, and/or not seeking diagnosis for a cancer such as pancreatic cancer.

A sample from a subject, e.g., a human or other mammalian subject, can be a sample of, e.g., blood, blood component (e.g., plasma, buffy coat), cfDNA (cell free DNA), ctDNA (circulating tumor DNA), stool, or tissue. In some particular embodiments, a sample is an excretion or bodily fluid of a subject (e.g., stool, blood, plasma, lymph, or urine of a subject) or a tissue sample. A sample from a subject can be a cell or tissue sample, e.g., a cell or tissue sample that is of a cancer or includes cancer cells, e.g., of a tumor or of a metastatic tissue. In various embodiments, a sample from a subject, e.g., a human or other mammalian subject, can be obtained by biopsy or surgery.

In various particular embodiments, a sample is a sample of cell-free DNA (cfDNA). cfDNA is typically found in biological fluids (e.g., plasma, serum, or urine) in short, double-stranded fragments. The concentration of cfDNA is typically low, but can significantly increase under particular conditions, including without limitation pregnancy, autoimmune disorder, myocardial infraction, and cancer. Circulating tumor DNA (ctDNA) is the component of circulating DNA specifically derived from cancer cells. ctDNA can be present in human fluids. For example in some instances, ctDNA can be found bound to and/or associated with leukocytes and erythrocytes. In some instances, ctDNA can be found not bound to and/or associated with leukocytes and erythrocytes. Various tests for detection of tumor-derived cfDNA are based on detection of genetic or epigenetic modifications that are characteristic of cancer (e.g., of a relevant cancer). Genetic or epigenetic modifications characteristic of cancer can include, without limitation, oncogenic or cancer-associated mutations in tumor-suppressor genes, activated oncogenes, hypermethylation, and/or chromosomal disorders. Detection of genetic or epigenetic modifications characteristic of cancer or pre-cancer can confirm that detected cfDNA is ctDNA.

cfDNA and ctDNA provide a real-time or nearly real-time metric of the methylation status of a source tissue. cfDNA and ctDNA have a half-life in blood of about 2 hours, such that a sample taken at a given time provides a relatively timely reflection of the status of a source tissue.

Various methods of isolating nucleic acids from a sample (e.g., of isolating cfDNA from blood or plasma) are known in the art. Nucleic acids can be isolated, e.g., without limitation, standard DNA purification techniques, by direct gene capture (e.g., by clarification of a sample to remove assay-inhibiting agents and capturing a target nucleic acid, if present, from the clarified sample with a capture agent to produce a capture complex, and isolating the capture complex to recover the target nucleic acid).

In certain embodiments, a sample may have a required minimum amount of DNA (e.g., cfDNA, gDNA) (e.g., DNA fragments) for later determining a methylation status. For example, in certain embodiments, a sample may be required to have at least 5 ng, at least 10 ng, at least 20 ng (or more) DNA.

Methods of Measuring Methylation Status

Methylation status can be measured by a variety of methods known in the art and/or by methods provided in this specification. Those of skill in the art will appreciate that a method for measuring methylation status can generally be applied to samples from any source and of any kind, and will further be aware of processing steps available to modify a sample into a form suitable for measurement by a given methodology.

In certain embodiments, the processing steps involve fragmenting or shearing DNA of the sample. For example, genomic DNA (e.g., gDNA) obtained from a cell, tissue, or other source may require fragmentation prior to sequencing. In certain embodiments, DNA may be fragmented prior to measurement of methylation status using a physical method (e.g., using an ultra-sonicator, a nebulizer technique, hydrodynamic shearing, etc.). In certain embodiments, DNA may be fragmented using an enzymatic method (e.g., using an endonuclease or a transposase). Certain samples, e.g., cfDNA samples, may not require fragmentation. cfDNA fragments are about 200bp in length and may be appropriate for certain methods provided herein. DNA fragments of about 100-1000 bp in length are suitable for analysis in certain NGS techniques described herein including, for example, Illumina® based techniques. Certain technologies may require DNA fragments of about 100-1000bp range. In contrast, DNA fragments of about 10kb or longer are suitable for long read sequencing technologies.

Methods of measuring methylation status include, without limitation, methods including whole genome bisulfite sequencing, targeted bisulfite sequencing, targeted enzymatic methylation sequencing, methylation-status-specific polymerase chain reaction (PCR), methods including mass spectrometry, methylation arrays, methods including methylation-specific nucleases, methods including mass-based separation, methods including target-specific capture (e.g., hybrid capture), and methods including methylation-specific oligonucleotide primers. Certain particular assays for methylation utilize a bisulfite reagent (e.g., hydrogen sulfite ions) or enzymatic conversion reagents (e.g., Tet methylcytosine dioxygenase 2).

Bisulfite reagents can include, among other things, bisulfite, disulfite, hydrogen sulfite, sodium metabisulphite, or combinations thereof, which reagents can be useful in distinguishing methylated and unmethylated nucleic acids. Bisulfite interacts differently with cytosine and 5-methylcytosine. In typical bisulfite-based methods, contacting of DNA (e.g., single stranded DNA, double stranded DNA) with bisulfite deaminates (e.g., converts) unmethylated cytosine to uracil, while methylated cytosine remains unaffected. Methylated cytosines, but not unmethylated cytosines, are selectively retained. Thus, in a bisulfite processed sample, uracil residues stand in place of, and thus provide an identifying signal for, unmethylated cytosine residues, while remaining (methylated) cytosine residues thus provide an identifying signal for methylated cytosine residues. Bisulfite processed samples can be analyzed, e.g., by next generation sequencing (NGS) or other methods disclosed herein.

In some embodiments, bisulfite processed samples may be treated using a bisulfite ratio of bisulfite to DNA that is at least. In certain embodiments, the bisulfite processed sample comprises single stranded DNA fragments or double stranded DNA fragments.

In some embodiments, bisulfite treatment includes subjecting DNA fragments (e.g., double stranded DNA) to one or more denaturation-conversion cycles in order to convert unmethylation cytosines to uracils in the DNA fragments. Denaturation converts double stranded DNA fragments in the sample to single stranded DNA fragments. Conversion changes the unmethylated cytosines of the single stranded DNA into uracils. In some embodiments, only one denaturation-conversion cycle are performed. In some embodiments, two, three, four, five, six, seven, eight, nine, ten, fifteen, twenty, or more denaturation-conversion cycles are performed. In some embodiments, the temperature of the denaturation step is performed at a temperature of about 80-100°C (e.g., about 90-97°C, e.g., about 96°C). In some embodiments, the denaturation step is performed for less than 10 minutes (e.g., less than 5 minutes, less than 5 minutes, less than 2 minutes, or less). In certain embodiments, the conversion step is performed for less than 2.5 hr (e.g., less than 2 hr, less than 1 hr, less than 30 minutes, less than 15 minutes, or less). In certain embodiments, the conversion step is performed at a temperature of 55 to 65°C. In certain embodiments, the converted DNA fragments may be stored at a temperature of about 4°C after performing the denaturation-conversion cycle(s). In some embodiments, bisulfite treatment may be applied prior to library preparation. In some embodiments, bisfulfite treatment may be applied after library preparation.

Enzymatic conversion reagents can include Tet methylcytosine dioxygenase 2 (TET2). TET2 oxidizes 5-methylcytosine and thus protects it from the consecutive deamination by APOBEC. APOBEC deaminates unmethylated cytosine to uracil, while oxidized 5-methylcytosine remains unaffected. Thus, in a TET2 processed sample, uracil residues stand in place of, and thus provide an identifying signal for, unmethylated cytosine residues, while remaining (methylated) cytosine residues thus provide an identifying signal for methylated cytosine residues. TET2 processed samples can be analyzed, e.g., by next generation sequencing (NGS). In certain embodiments, APOBEC refers to a member (or plurality of members) of the Apolipoprotein B mRNA Editing Catalytic Polypeptide-like (APOBEC) family. In certain embodiments, APOBEC may refer to APOBEC-1, APOBEC-2, APOBEC-3A, APOBEC-3B, APOBEC-3C, APOBEC-3D, APOBEC-3E, APOBEC-3F, APOBEC-3G. APOBEC-3H, APOBEC-4, and/or Activation-induced (cytidine) deaminase (AID).

Methods of measuring methylation status can include, without limitation, massively parallel sequencing (e.g., next-generation sequencing) to determine methylation state, e.g., sequencing by- synthesis, real-time (e.g., single-molecule) sequencing, bead emulsion sequencing, nanopore sequencing, or other sequencing techniques known in the art. In some embodiments, a method of measuring methylation status can include whole-genome sequencing, e.g., measuring whole genome methylation status from bisulfite or enzymatically treated material with base-pair resolution.

In some embodiments, a method of measuring methylation status includes reduced representation bisulfite sequencing e.g., utilizing use of restriction enzymes to measure methylation status of high CpG content regions from bisulfite or enzymatically treated material with base-pair resolution.

In some embodiments, a method of measuring methylation status can include targeted sequencing e.g., measuring methylation status of pre-selected genomic location from bisulfite or enzymatically treated material with base-pair resolution.

In some embodiments, the pre-selection (capture) (e.g., enrichment) of regions of interest (e.g., DMRs) can be done by complementary *in vitro* synthesized oligonucleotide sequences (e.g., capture baits/probes). Capture probes (e.g., oligonucleotide capture probes, oligonucleotide capture baits) are useful in targeted sequencing (e.g., NGS) techniques to enrich for particular regions of interest in an oligonucleotide (e.g., DNA) sequence. For example, enrichment of target regions is useful when sequences of particular pre-determined regions of DNA are sequenced. In certain embodiments, capture probes are about 10 to 1000bp long (e.g., about 10 to about 200bp long) (e.g., about 120bp long). In certain embodiments, one or more capture probes are targeted to capture a region of interest (e.g., a genomic marker) corresponding to one or more methylation loci (e.g., methylation loci comprising at least a portion of one or more DMRs, e.g., as found in FIG. 2 and/or FIGs. 3A-3G). In certain embodiments, capture probes are targeted to methylation loci that are hypomethylated or hypermethylated. For example, a capture probe may be targeted to a particular methylation loci. However, if fragments of DNA corresponding to a methylation loci are converted (e.g., bisulfite or enzymatic converted) prior to enrichment using a capture probe, the sequence of the converted DNA fragments will change as described herein due to particular cytosine residues being unmethylated. Therefore, targeting an unconverted DNA region may result in some mismatches if cytosines are hypomethylated. Though capture probe-target sequence hybridization may tolerate some mismatches, a second probe may be required to enrich for DNA regions which are hypomethylated.

In certain embodiments, capture probes are evaluated (e.g., prior to sequencing) for their ability to target multiple regions of the genome of interest. For example, when designing a capture probe to target a particular region of interest (e.g., a DMR), the ability for a capture probe to target multiple regions of the genome may be considered. As discussed herein, mismatches in pairing (e.g., non-Watson-Crick pairing) allow for capture probes to hybridize to other, unintended regions of a genome. In addition, a particular target sequence may be repeated elsewhere in a genome. Repeat sequences are common for sequences that are highly repetitive. In certain embodiments, capture probes are designed such that they only target a few similar regions of the genome. In certain embodiments, capture probes may hybridize to 500 or fewer, 100 or fewer, 50 or fewer, 10 or fewer, 5 or fewer similar regions in a genome. In certain embodiments, a similar region to the target of region of interest is calculated using a 24bp window moving around a genome and matching the region of the window to a reference sequence according to sequence order similarity. Other size windows and/or techniques may be used.

For example, hybrid-capture of one or more DNA fragments (e.g., ctDNA, fragmented gDNA) may be performed using capture probes targeted to predetermined regions of interested of a genome. In certain embodiments, capture probes target at least 2 (e.g, at least 3, 4, 5, 6, 7, 8, 9, 10, 11, 12, 13, 14, 15, 16, 17, 18, 19, 20, 21, 22, 23, 24, 25, 50, 75, 100, 150, or more) predetermined regions of interest (e.g., genomic markers, e.g., DMRs). In certain embodiments, the capture probes overlap. In certain embodiments, the overlapping probes overlap at least 10%, at least 20%, at least 30%, at least 40%, at least 50%, at least 60% or more.

In certain embodiments, the capture probes are nucleic acid probes (e.g., DNA probes, RNA probes). In some embodiments, a method may also include identifying mutated regions (e.g., individual nucleotide bases) using targeted sequencing e.g., determining the presence of a mutation in one or more pre-selected genomic locations (e.g., a genomic marker, e.g., a mutation marker). In certain embodiments, mutations may also be identified from bisulfite or enzymatically treated DNA with base-pair resolution.

In some embodiments, a method for measuring methylation status can include Illumina Methylation Assays e.g., measuring over 850,000 methylation sites quantitatively across a genome at single-nucleotide resolution.

Various methylation assay procedures can be used in conjunction with bisulfite treatment to determine methylation status of a target sequence such as a DMR. Such assays can include, among others, Methylation-Specific Restriction Enzyme qPCR, sequencing of bisulfite-treated nucleic acid, PCR (e.g., with sequence-specific amplification), Methylation Specific Nuclease-assisted Minor-allele Enrichment PCR, and Methylation-Sensitive High Resolution Melting. In some embodiments, DMRs are amplified from converted (e.g., bisulfite or enzyme converted) DNA fragments for library preparation.

In some embodiments, a sequencing library may be prepared using converted (e.g., bisulfite or enzyme converted) oligonucleotide fragments (e.g., cfDNA, gDNA fragments, synthetic nucleotide sequences, etc.) according to, e.g., an Illumina protocol, an Accel-NGS® Methyl-Seq DNA Library Kit (Swift Bioscience) protocol, a transpose-based Nextera XT protocol, or the like. In some embodiments, the oligonucleotide fragments are DNA fragments which have been converted (e.g., bisulfite or enzyme converted). In certain embodiments, DNA fragments used in preparation of a sequencing library may be single stranded DNA fragments or double stranded DNA fragments. In certain embodiments, a library may be prepared by attaching adapters to DNA fragments. Adapters contain short (e.g., about 100 to about 1000bp) sequences (e.g., oligonucleotide sequences) that allow oligonucleotide fragments of a library (e.g., a DNA library) to bind to and generate clusters on a flow cell used in, for example, next generation sequencing (NGS). Adapters may be ligated to library fragments prior to NGS. In certain embodiments, a ligase enzyme covalently links the adapter and library fragments. In certain embodiments, adapters are attached to either one or both of the 5' and 3' ends of converted DNA fragments. In certain embodiments, the attaching step is performed such that at least 40%, at least 50%, at least 60%, at least 70% of the converted DNA fragments are attached to adapter. In certain embodiments, the attaching step is performed such that at least 40%, at least 50%, at least 60%, at least 70% of the converted DNA fragments have an adapter attached at both the 5' and 3' ends

In certain embodiments, adapters used herein contain a sequence of oligonucleotides that aid in sample identification. For example, in certain embodiments, adapters include a sample index. A sample index is a short sequence (e.g., about 8 to about 10 bases) of nucleic acids (e.g., DNA, RNA) that serve as sample identifiers and allow for, among other things, multiplexing and/or pooling of multiple samples in a single sequencing run and/or on a flow cell (e.g., used in a NGS technique). In certain embodiments, an adapter at a 5' end, a 3' end, or both of a converted single stranded DNA fragment includes a sample index. In certain embodiments, an adapter sequence may include a molecular barcode. A molecular barcode may serve as a unique molecular identifier to identify a target molecule during, for example, DNA sequencing. In certain embodiments, DNA barcodes may be randomly generated. In certain embodiments, DNA barcodes may be predetermined or predesigned. In certain embodiments, the DNA barcodes are different on each DNA fragment. In certain embodiments, the DNA barcodes may be the same for two single stranded DNA fragments that are not complementary to one another (e.g., in a Watson-Crick pair with each other) in the biological sample. In certain embodiments, DNA fragments may be amplified (e.g., using PCR) after ligation of adapters to DNA fragments. In certain embodiments, at least 40% (e.g., at least at least 50%, at least 60%, at least 70%) of the converted DNA fragments have an adapter attached at both the 5' and 3' ends.

In certain embodiments, high-throughput and/or next-generation sequencing (NGS) techniques are used to achieve base-pair level resolution of an oligonucleotide (e.g., a DNA) sequence, permitting analysis of methylation status and/or identification of mutations. For example, in certain embodiments, NGS may include single-end or paired-end sequencing. In single-end sequencing, a technique reads a sequenced fragment in one direction - from one end of a fragment to the opposite end of the fragment. In certain embodiments, this produces a single DNA sequence that then may be aligned to a reference sequence. In paired-end sequencing, a sequenced fragment is read in a first direction from one end of the fragment to the opposite end of the fragment. The sequenced fragment may be read until a specified read length is reached. Then, the sequenced fragment is read in a second direction, which is opposite to the first direction. In certain embodiments, having multiple read pairs may help to improve read alignment and/or identify mutations (e.g., insertions, deletions, inversion, etc.) that may not be detected by single-end reading.

Another method, that can be used for methylation detection includes PCR amplification with methylation-specific oligonucleotide primers (MSP methods), e.g., as applied to bisulfite-treated sample (see, e.g., Herman 1992 Proc. Natl. Acad. Sci. USA 93: 9821-9826, which is herein incorporated by reference with respect to methods of determining methylation status). Use of methylation-status-specific oligonucleotide primers for amplification of bisulfite-treated DNA allows differentiation between methylated and unmethylated nucleic acids. Oligonucleotide primer pairs for use in MSP methods include at least one oligonucleotide primer capable of hybridizing with sequence that includes a methylation site, e.g., a CpG site. An oligonucleotide primer that includes a T residue at a position complementary to a cytosine residue will selectively hybridize to templates in which the cytosine was unmethylated prior to bisulfite treatment, while an oligonucleotide primer that includes a G residue at a position complementary to a cytosine residue will selectively hybridize to templates in which the cytosine was methylated cytosine prior to bisulfite treatment. MSP results can be obtained with or without sequencing amplicons, e.g., using gel electrophoresis. MSP (methylation-specific PCR) allows for highly sensitive detection (detection level of 0.1% of the alleles, with full specificity) of locus-specific DNA methylation, using PCR amplification of bisulfite-converted DNA.

Another method that can be used to determine methylation status after bisulfite treatment of a sample is Methylation-Sensitive High Resolution Melting (MS-HRM) PCR (see, e.g., Hussmann 2018 Methods Mol Biol. 1708:551-571, which is herein incorporated by reference with respect to methods of determining methylation status). MS-HRM is an in-tube, PCR-based method to detect methylation levels at specific loci of interest based on hybridization melting. Bisulfite treatment of the DNA prior to performing MS-HRM ensures a different base composition between methylated and unmethylated DNA, which is used to separate the resulting amplicons by high resolution melting. A unique primer design facilitates a high sensitivity of the assays enabling detection of down to 0.1-1% methylated alleles in an unmethylated background. Oligonucleotide primers for MS-HRM assays are designed to be complementary to the methylated allele, and a specific annealing temperature enables these primers to anneal both to the methylated and the unmethylated alleles thereby increasing the sensitivity of the assays.

Another method that can be used to determine methylation status after bisulfite treatment of a sample is Quantitative Multiplex Methylation-Specific PCR (QM-MSP). QM-MSP uses methylation specific primers for sensitive quantification of DNA methylation (see, e.g., Fackler 2018 Methods Mol Biol. 1708:473-496, which is herein incorporated by reference with respect to methods of determining methylation status). QM-MSP is a two-step PCR approach, where in the first step, one pair of gene-specific primers (forward and reverse) amplifies the methylated and unmethylated copies of the same gene simultaneously and in multiplex, in one PCR reaction. This methylation-independent amplification step produces amplicons of up to $10^9$ copies per $\mu$L after 36 cycles of PCR. In the second step, the amplicons of the first reaction are quantified with a standard curve using real-time PCR and two independent fluorophores to detect methylated/unmethylated DNA of each gene in the same well (e.g., 6FAM and VIC). One methylated copy is detectable in 100,000 reference gene copies.

Another method that can be used to determine methylation status after bisulfite treatment of a sample is Methylation Specific Nuclease-assisted Minor-allele Enrichment (MS-NaME) (see, e.g., Liu 2017 Nucleic Acids Res. 45(6):e39, which is herein incorporated by reference with respect to methods of determining methylation status). Ms-NaME is based on selective hybridization of probes to target sequences in the presence of DNA nuclease specific to double-stranded (ds) DNA (DSN), such that hybridization results in regions of double-stranded DNA that are subsequently digested by the DSN. Thus, oligonucleotide probes targeting unmethylated sequences generate local double stranded regions resulting to digestion of unmethylated targets; oligonucleotide probes capable of hybridizing to methylated sequences generate local double-stranded regions that result in digestion of methylated targets, leaving methylated targets intact. Moreover, oligonucleotide probes can direct DSN activity to multiple targets in bisulfite-treated DNA, simultaneously. Subsequent amplification can enrich non-digested sequences. Ms-NaME can be used, either independently or in combination with other techniques provided herein.

Another method that can be used to determine methylation status after bisulfite treatment of a sample is Methylation-sensitive Single Nucleotide Primer Extension (Ms-SNuPE™) (see, e.g., Gonzalgo 2007 Nat Protoc. 2(8):1931-6, which is herein incorporated by reference with respect to methods of determining methylation status). In Ms-SNuPE, strand-specific PCR is performed to generate a DNA template for quantitative methylation analysis using Ms-SNuPE. SNuPE is then performed with oligonucleotide(s) designed to hybridize immediately upstream of the CpG site(s) being interrogated. Reaction products can be electrophoresed on polyacrylamide gels for visualization and quantitation by phosphor-image analysis. Amplicons can also carry a directly or indirectly detectable labels such as a fluorescent label, radionuclide, or a detachable molecule fragment or other entity having a mass that can be distinguished by mass spectrometry. Detection may be carried out and/or visualized by means of, e.g., matrix assisted laser desorption/ionization mass spectrometry (MALDI) or using electron spray mass spectrometry (ESI).

Certain methods that can be used to determine methylation status after bisulfite treatment of a sample utilize a first oligonucleotide primer, a second oligonucleotide primer, and an oligonucleotide probe in an amplification-based method. For instance, the oligonucleotide primers and probe can be used in a method of real-time polymerase chain reaction (PCR) or droplet digital PCR (ddPCR). In various instances, the first oligonucleotide primer, the second oligonucleotide primer, and/or the oligonucleotide probe selectively hybridize methylated DNA and/or unmethylated DNA, such that amplification or probe signal indicate methylation status of a sample.

Other bisulfite-based methods for detecting methylation status (e.g., the presence of level of 5-methylcytosine) are disclosed, e.g., in Frommer (1992 Proc Natl Acad Sci U S A. 1 ;89(5):1827-31, which is herein incorporated by reference with respect to methods of determining methylation status).

In certain MSRE-qPCR embodiments, the amount of total DNA is measured in an aliquot of sample in native (e.g., undigested) form using, e.g., real-time PCR or digital PCR.

Various amplification technologies can be used alone or in conjunction with other techniques described herein for detection of methylation status. Those of skill in the art, having reviewed the present specification, will understand how to combine various amplification technologies known in the art and/or described herein together with various other technologies for methylation status determination known in the art and/or provided herein. Amplification technologies include, without limitation, PCR, e.g., quantitative PCR (qPCR), real-time PCR, and/or digital PCR. Those of skill in the art will appreciate that polymerase amplification can multiplex amplification of multiple targets in a single reaction. PCR amplicons are typically 100 to 2000 base pairs in length. In various instances, an amplification technology is sufficient to determine methylations status.

Digital PCR (dPCR) based methods involve dividing and distributing a sample across wells of a plate with 96-, 384-, or more wells, or in individual emulsion droplets (ddPCR) e.g., using a microfluidic device, such that some wells include one or more copies of template and others include no copies of template. Thus, the average number of template molecules per well is less than one prior to amplification. The number of wells in which amplification of template occurs provides a measure of template concentration. If the sample has been contacted with MSRE, the number of wells in which amplification of template occurs provides a measure of the concentration of methylated template.

In various embodiments a fluorescence-based real-time PCR assay, such as MethyLight™, can be used to measure methylation status (see, e.g., Campan 2018 Methods Mol Biol. 1708:497-513, which is herein incorporated by reference with respect to methods of determining methylation status). MethyLight is a quantitative, fluorescence-based, real-time PCR method to sensitively detect and quantify DNA methylation of candidate regions of the genome. MethyLight is uniquely suited for detecting low-frequency methylated DNA regions against a high background of unmethylated DNA, as it combines methylation-specific priming with methylation-specific fluorescent probing. Additionally, MethyLight can be combined with Digital PCR, for the highly sensitive detection of individual methylated molecules, with use in disease detection and screening.

Real-time PCR-based methods for use in determining methylation status typically include a step of generating a standard curve for unmethylated DNA based on analysis of external standards. A standard curve can be constructed from at least two points and can permit comparison of a real-time Ct value for digested DNA and/or a real-time Ct value for undigested DNA to known quantitative standards. In particular instances, sample Ct values can be determined for MSRE-digested and/or undigested samples or sample aliquots, and the genomic equivalents of DNA can be calculated from the standard curve. Ct values of MSRE-digested and undigested DNA can be evaluated to identify amplicons digested (e.g., efficiently digested; e.g., yielding a Ct value of 45). Amplicons not amplified under either digested or undigested conditions can also be identified. Corrected Ct values for amplicons of interest can then be directly compared across conditions to establish relative differences in methylation status between conditions. Alternatively or additionally, delta-difference between the Ct values of digested and undigested DNA can be used to establish relative differences in methylation status between conditions.

In certain particular embodiments, targeted bisulfite sequencing (e.g., using hybrid capture) among other techniques, can be used to determine the methylation status of a methylation biomarker for a disease and/or condition. For example, a pancreatic cancer methylation biomarker that is or includes a single methylation locus. In certain particular embodiments, targeted bisulfite sequencing, among other techniques, can be used to determine the methylation status of a methylation biomarker that is or includes two or more methylation loci.

Those of skill in the art will appreciate that in embodiments in which a plurality of methylation loci (e.g., a plurality of DMRs) are analyzed for methylation status in a method of screening for pancreatic cancer provided herein, methylation status of each methylation locus can be measured or represented in any of a variety of forms, and the methylation statuses of a plurality of methylation loci (preferably each measured and/or represented in a same, similar, or comparable manner) be together or cumulatively analyzed or represented in any of a variety of forms. In various embodiments, methylation status of each methylation locus can be measured as methylation portion. In various embodiments, methylation status of each methylation locus can be represented as the percentage value of methylated reads from total sequencing reads compared against reference sample. In various embodiments, methylation status of each methylation locus can be represented as a qualitative comparison to a reference, e.g., by identification of each methylation locus as hypermethylated or hypomethylated. In some embodiments in which a single methylation locus is analyzed, hypermethylation of the single methylation locus constitutes a diagnosis that a subject is suffering from or possibly suffering from a condition (e.g., cancer) (e.g., pancreatic cancer), while absence of hypermethylation of the single methylation locus constitutes a diagnosis that the subject is likely not suffering from a condition. In some embodiments, hypermethylation of a single methylation locus (e.g., a single DMR) of a plurality of analyzed methylation loci constitutes a diagnosis that a subject is suffering from or possibly suffering from the condition, while the absence of hypermethylation at any methylation locus of a plurality of analyzed methylation loci constitutes a diagnosis that a subject is likely not suffering from the condition. In some embodiments, hypermethylation of a determined percentage (e.g., a predetermined percentage) of methylation loci (e.g., at least 10% (e.g., at least 10%, at least 20%, at least 30%, at least 40%, at least 50%, at least 60%, at least 70%, at least 80%, at least 90%, or 100%)) of a plurality of analyzed methylation loci constitutes a diagnosis that a subject is suffering from or possibly suffering from the condition, while the absence of hypermethylation of a determined percentage (e.g., a predetermined percentage) of methylation loci (e.g., at least 10% (e.g., at least 10%, at least 20%, at least 30%, at least 40%, at least 50%, at least 60%, at least 70%, at least 80%, at least 90%, or 100%)) of a plurality of analyzed methylation loci constitutes a diagnosis that a subject is not likely suffering from the condition. In some embodiments, hypermethylation of a determined number (e.g., a predetermined number) of methylation loci (e.g., at least 1, 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) of a plurality of analyzed methylation loci (e.g 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) constitutes a diagnosis that a subject is suffering from or possibly suffering from the condition, while the absence of hypermethylation of a determined number (e.g., a predetermined number) of methylation loci (e.g., at least 1, 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) of a plurality of analyzed methylation loci (e.g., 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) constitutes a diagnosis that a subject is not likely suffering from the condition.

In some embodiments, methylation status of a plurality of methylation loci (e.g., a plurality of DMRs) is measured qualitatively or quantitatively and the measurement for each of the plurality of methylation loci are combined to provide a diagnosis. In some embodiments, the quantitatively measured methylation status of each of a plurality of methylation loci is individually weighted, and weighted values are combined to provide a single value that can be comparative to a reference in order to provide a diagnosis.

In some embodiments, methylation status may include determination of methylated and/or unmethylated reads mapped to a genomic region (e.g., a DMR). For example, when using particular sequencing technologies as disclosed herein (e.g., NGS, whole genome bisulfite sequencing, etc.), sequence reads are produced. A sequence read is an inferred sequence of base pairs (e.g., a probabilistic sequence) corresponding to all or part of a sequenced oligonucleotide (e.g., DNA) fragment (e.g., cfDNA fragments, gDNA fragments). In certain embodiments, sequence reads may be mapped (e.g., aligned) to a particular region of interest using a reference sequence (e.g., a bisulfite converted reference sequence) in order to determine if there are any alterations or variations in a read. Alterations may include methylation and/or mutations. A region of interest may include one or more genomic markers including a methylation marker (e.g., a DMR), a mutation marker, or other marker as disclosed herein.

For example, in the case of bisulfite or enzymatically treated DNA fragments, treatment converts unmethylated cytosines to uracils, while methylated cytosines are not converted to uracils. Accordingly, a sequence read produced for a DNA fragment that has methylated cytosines will be different from a sequence read produced for the same DNA fragment that does not have methylated cytosine. Methylation at sites where a cytosine nucleotide is followed by a guanine nucleotide (e.g., CpG sites) may be of particular interest.

Quality Control Protocol

In certain embodiments, quality control steps may be implemented. Quality control steps are used to determine whether or not particular steps or processes were conducted within particular parameters. In certain embodiments, quality control steps may be used to determine the validity of results of a given analysis. In addition or alternatively, quality control steps may be used to determine sequenced data quality. For example, quality control steps may be used to determine read coverage of one or more regions of DNA. Quantitative metrics for quality control include, but are not limited to AT dropout rate, GC dropout rate, bisulfite conversion rate (e.g., bisulfite conversion efficiency), and the like. Failure to meet a threshold quality control condition (e.g., a minimum conversion rate, a maximum CG dropout rate, etc.) may indicate, for example, that one or more of the conversion steps were not performed within appropriate parameters.

For example, in the methods described herein, various steps of a conversion protocol may be optimized to decrease AT and/or GC dropout rate. As is understood by those of skill in the art, AT and GC dropout metrics indicate the degree of inadequate coverage of a particular target region based on its AT or GC content. In certain embodiments, samples having a low GC dropout rate is useful in identifying which samples were processed appropriately. For example, a GC dropout rate found to be less than 10%, less than 9%, less than 8%, less than 7%, less than 6%, less than 5%, less than 4%, or less may be useful in identifying appropriately processed samples.

In certain embodiments, a quality control step may involve determining an on and/or off-target ratio. Sequence reads that align to a region of interest (e.g., a DMR) are considered to be on-target, while sequence reads that do not align to the region of interest (e.g., a DMR) are considered to be off-target. In certain embodiments, the on-target ratio is represented as a percentage of on-target bases to the total number of aligned bases. In certain embodiments, the on-target ratio is represented as a percentage of on-target and near-target bases to the total number of aligned bases. Near-target bases may be a base within a certain number of bases (e.g., within 500bp, within 200bp, within 100bp) of the target region. In certain embodiments, the on-target ratio is at least 10%, least 20%, least 30%, least 40%, least 50%, least 60%, least 70%, at least 80%, at least 90%, at least 95%, at least 99% or more for a sequencing experiment to pass quality control. In certain embodiments, the off-target ratio is represented as a percentage of off-target bases to the total number of aligned bases. In certain embodiments, an off-target ratio is less than 95%, less than 90%, less than 85%, less than 80%, less than 70%, less than 60%, less than 50%, less than 40%, less than 30%, less than 20%, less than 10%, less than 1% for a sequencing experiment to pass quality control.

In certain embodiments, a quality control step may include determining quality scores for mapped sequence reads. A quality score is a value which quantifies a probability that a sequence read is incorrectly mapped. For example, when mapping short or repetitive sequences, it is possible that a sequence will be mapped to multiple places in a reference genome. The quality score considers the best alignment of the sequence read to the reference genome as compared to other possible alignments of the sequence read to the reference genome. In certain embodiments, the quality score is a Mapping Quality (MAPQ) score. The MAPQ is the negative, log-scaled probability that a read is misaligned. A high score indicates a high confidence that a read is aligned correctly, while a low score indicates a low confidence that a read is aligned correctly. In certain embodiments, the MAPQ score may be calculated using the following equation:

$$\text{MAPQ score} = -10 \log_{10} \Pr\{ \text{mapping position is wrong} \}.$$

In certain embodiments, the MAPQ score is rounded to the nearest integer. In certain embodiments Pr is a probability that the sequence read is incorrectly mapped as obtained from an alignment (e.g., mapping) tool. In certain embodiments, the scaling factor is 1 (instead of 10), or another number.

Artificial Spike-in Control

Control nucleic acid (e.g., DNA) molecules (e.g., "spike-in controls") may be used to evaluate or estimate conversion efficiency of unmethylated and methylated cytosines to uracils. Control nucleic acid molecules may be used in sequencing methods involving conversion (e.g., bisulfite or enzymatic conversion) of DNA samples.

When DNA is subjected to conversion (e.g., bisulfite or enzymatic conversion) as described herein, conversion may be incomplete. That is, some number of unmethylated cytosines may not be converted to uracils. If the conversion is not complete such that unmethylated cytosines are not mostly converted, the unconverted unmethylated cytosines may be identified as methylated when the DNA sequenced. Accordingly, in order to determine whether or not bisulfite conversion is complete, a control DNA molecule may be subjected to conversion along with DNA fragments from a sample. In certain embodiments, sequencing the converted control DNA molecules (e.g., using an NGS technique as described herein) generates a plurality of control sequence reads. Control sequence reads may be used to determine conversion rates of unmethylated and/or methylated cytosines to uracils.

Prior techniques did not recognize that controls (e.g., a control DNA molecule) were useful to include in each sample. Rather, they presumed that conversion efficiencies remained relatively consistent between samples for a given run. However, the inventors have identified that conversion rate of unmethylated cytosines to uracils in DNA fragments may vary significantly from on sample to another. For example, conversion efficiency may range from 10% to 110% within a single batch of processed samples. Note, there can be overconversion such that conversion efficiency can be greater than 100%, e.g., the conversion efficiency is 110% when 10% of the methylated cytosine gets converted. In certain embodiments, the conversion efficiency ranges from 30% to 110%. In other embodiments, the conversion efficiency ranges from 50% to 100%.

In certain embodiments, a control DNA molecule may be added to a sample after fragmentation and before conversion using e.g., bisulfite or enzymatic reagents. In certain embodiments, a plurality (e.g., two, three, four or more) control DNA sequences may be added to DNA fragments of a sample. A control DNA molecule may be a known sequence. For example, the sequence, number of methylated bases, and number of unmethylated bases of the control sequence had been determined prior to addition of the control DNA molecule to the sample. In certain embodiments, a control sequence may be a DNA sequence which is produced *in vitro* to contain artificially methylated or unmethylated nucleotides (e.g., methylated cytosines). In certain embodiments, a control sequence may be a DNA sequence which is produced to contain completely unmethylated DNA nucleotides.

A high conversion efficiency of the spike-in control sequence may be used to infer the conversion efficiency of a DNA fragments undergoing the same conversion process as a spike-in control. For example, deamination of at least at least 98% of unmethylated cytosines in the unmethylated spike-in control DNA sequence indicates that conversion efficiency is high and that a sample may pass a quality control assessment. In certain embodiments, at least 90%, at least 95%, at least 96%, at least 97%, at least 98%, at least 99% of unmethylated cytosines of a plurality of DNA fragments of a control DNA sequence are converted into uracils. A high conversion efficiency is important as it is ideal for all (or nearly all) of the unmethylated cytosines to be converted to uracils when subjecting DNA to bisulfite or enzymatic treatments. As described above, unconverted, unmethylated cytosines may serve as a source of noise in the data.

In addition, conversion of methylated cytosines to uracils is undesirable when DNA is treated using a conversion process. Conversion of methylated cytosines of a spike-in control is indicative that methylated cytosines have been converted to uracils in a DNA sample subjected to the same treatment as the methylated spike-in control. Methylated cytosines in a methylated spike-in control should not convert to uracils. For the same reasons as described above, methylated cytosines being converted to uracils may result in misidentification of purportedly unmethylated cytosines during methylation analysis. In certain embodiments, at most 5%, at most 4%, at most 3%, at most 2% or at most 1% of methylated cytosines of a plurality of DNA fragments of a control DNA sequence are converted into uracils. For example, deamination of at most 2% of methylated cytosines in a methylated spike-in control DNA sequence indicates that conversion efficiency is high and that a sample may pass a quality control assessment.

Applications

Methods and compositions of the present disclosure can be used in any of a variety of applications. For example, methods and compositions of the present disclosure can be used to screen, or aid in screening for a condition (e.g., pancreatic cancer). In various instances, screening using methods and compositions of the present disclosure can detect any stage of pancreatic cancer, including without limitation early-stage pancreatic cancer. In some embodiments, screening using methods and compositions of the present disclosure is applied to individuals 40 years of age or older, e.g.,

40, 45, 50, 55, 60, 65, 70, 75, 80, 85, or 90 years or older. In particular, individuals 40 years of age or older are of interest for pancreatic cancer screening. In some embodiments, screening using methods and compositions of the present disclosure is applied to individuals 18 years of age or older, e.g., 18, 20, 25, 30, 35, 40, 45, 50, 55, 60, 65, 70, 75, 80, 85, or 90 years or older. In some embodiments, screening using methods and compositions of the present disclosure is applied to individuals 18 to 40 years of age. In various embodiments, screening using methods and compositions of the present disclosure is applied to individuals experiencing abdominal pain or discomfort, e.g., experiencing undiagnosed or incompletely diagnosed abdominal pain or discomfort. In various embodiments, screening using methods and compositions of the present disclosure is applied to individuals experiencing no symptoms likely to be associated with a cancer. Thus, in certain embodiments, screening using methods and compositions of the present disclosure is fully or partially preventative or prophylactic, at least with respect to later or non-early stages of cancer.

In various embodiments, cancer screening using methods and compositions of the present disclosure can be applied to an asymptomatic human subject. In particular, a subject can be referred to as "asymptomatic" if the subject does not report, and/or demonstrate by non-invasively observable indicia (e.g., without one, several, or all of device-based probing, tissue sample analysis, bodily fluid analysis, surgery, or cancer screening), sufficient characteristics of the condition to support a medically reasonable suspicion that the subject is likely suffering from the condition.

Those of skill in the art will appreciate that regular, preventative, and/or prophylactic screening for pancreatic cancer improves diagnosis. Generally, and particularly in embodiments in which screening in accordance with the present disclosure is carried out annually, and/or in which a subject is asymptomatic at time of screening, methods and compositions of the present invention are especially likely to detect early stage pancreatic cancer.

In various embodiments, pancreatic cancer screening in accordance with the present disclosure is performed once for a given subject or multiple times for a given subject. In various embodiments, pancreatic cancer screening in accordance with the present disclosure is performed on a regular basis, e.g., every six months, annually, every two years, every three years, every four years, every five years, or every ten years.

In various embodiments, screening using methods and compositions disclosed herein will provide a diagnosis of a condition (e.g., a class of pancreatic cancer). Pancreatic cancer sub-categorization on a molecular level could help with better pre-treatment stratification of the patients and could help to direct the treatment regiments, which in return could lead to better outcomes. This has been shown to be the case for other cancer types such as breast cancer. Traditionally, molecular subtyping has been done on whole-genome sequencing and copy number variation identification, based on patterns of chromosomal structural variation with potential clinical utility. DNA methylation gives another layer for sub-categorization to the patients by providing cell-of-origin type of signature, that could identify different cellular origin of the cancer.

For the avoidance of any doubt, those of skill in the art will appreciate from the present disclosure that methods and compositions for pancreatic cancer diagnosis of the present specification are at least for in vitro use. Accordingly, all aspects and embodiments of the present disclosure can be performed and/or used at least in vitro.

Kits

The present disclosure includes, among other things, kits including one or more compositions for use in screening as provided herein, optionally in combination with instructions for use thereof in screening (e.g., screening for pancreatic cancer and/or other diseases or conditions associated with an aberrant methylation status, e.g., neurodegenerative diseases, gastrointestinal disorders, and the like). In various embodiments, a kit for screening a diseases or conditions associated with an aberrant methylation status can include one or more oligonucleotide probes (e.g., one or more biotinylated oligonucleotide probes). In certain embodiments, the kit for screening optionally includes one or more bisulfite conversion reagents as disclosed herein. In certain embodiments, the kit for screening optionally includes one or more enzymatic conversion reagents as disclosed herein. In certain embodiments, the kit for screening may include one or more adapters as described herein. In certain embodiments, the kit may include one or more reagents used in library preparation. In certain embodiments, the kit may include software (e.g., for analyzing methylation status of DMRs).

Elements of different implementations described herein may be combined to form other implementations not specifically set forth above. Elements may be left out of the processes, computer programs, databases, etc. described herein without adversely affecting their operation. In addition, the logic flows depicted in the figures do not require the particular order shown, or sequential order, to achieve desirable results. Various separate elements may be combined into one or more individual elements to perform the functions described herein.

Throughout the description, where apparatus and systems are described as having, including, or comprising specific components, or where processes and methods are described as having, including, or comprising specific steps, it is contemplated that, additionally, there are apparatus, and systems of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

While the invention has been particularly shown and described with reference to specific preferred embodiments, it should be understood by those skilled in the art that various changes in form and detail may be made therein without departing from the spirit and scope of the invention as defined by the appended claims.

**OTHER EMBODIMENTS**

While we have described a number of embodiments, it is apparent that our basic disclosure and examples may provide other embodiments that utilize or are encompassed by the compositions and methods described herein. Therefore, it will be appreciated that the scope of is to be defined by that which may be understood from the disclosure and the appended claims rather than by the specific embodiments that have been represented by way of example.

**Claims**

1. A method of identifying pancreatic cancer in a human subject, the method comprising:

   determining a methylation status of each of one or more markers identified in deoxyribonucleic acid (DNA) from a sample obtained from the subject,
   comparing the data obtained with reference values obtained from healthy individuals, and
   identifying pancreatic cancer in the subject based at least in part on the determined methylation status of each of the one or more markers as compared to the reference sample,
   wherein each of the one or more markers is a methylation locus comprising at least a portion of a differentially methylated region (DMR) selected from the DMRs listed in Table 1 or comprising at least a single differentially methylated CpG site (DMC) within one or more of the DMRs listed in the following Table 1, wherein each said portion comprises at least one (1) CpG and each said methylation locus has a length equal to or less than 302 bp:

| Symbol | Chr | start | end | SEQUENCE ID NO. |
|---|---|---|---|---|
| PTPRN; PTPRN | chr2 | 219309329 | 219309630 | 1 |
| RYR2 | chr1 | 237042549 | 237042850 | 2 |
| · | chr10 | 100659710 | 100660011 | 3 |
| GPR26 | chr10 | 123665959 | 123666260 | 4 |
| · | chr5 | 1875622 | 1875923 | 5 |
| AC003986.5; FERD3L | chr7 | 19145188 | 19145489 | 6 |
| AC01 0729.1; SOX11 | chr2 | 5695829 | 5696130 | 7 |
| TWIST1 | chr7 | 19106259 | 19106560 | 8 |
| RYR2 | chr1 | 237042500 | 237042801 | 9 |
| RP11-572N21.1; SP9 | chr2 | 174334950 | 174335251 | 10 |
| THBS4; THBS4 | chr5 | 80034970 | 80035271 | 11 |
| MDGA2 | chr14 | 47675647 | 47675948 | 12 |
| DBX1 | chr11 | 20156156 | 20156457 | 13 |
| · | chr7 | 35257207 | 35257508 | 14 |
| TMEM132D | chr12 | 129903539 | 129903840 | 15 |
| GDF6; GDF6 | chr8 | 96159449 | 96159750 | 16 |
| TWIST1; TWIST1 | chr7 | 19117490 | 19117791 | 17 |
| NPBWR1 | chr8 | 52939320 | 52939621 | 18 |
| · | chr13 | 112057477 | 112057778 | 19 |

(continued)

| Symbol | Chr | start | end | SEQUENCE ID NO. |
|---|---|---|---|---|
| MDGA2 | chr14 | 47675755 | 47676056 | 20 |
| SORCS3; SORCS3 | chr10 | 104640972 | 104641273 | 21 |
| PEX5L; PEX5L | chr3 | 180036595 | 180036896 | 22 |
| AC009487.5; SLC4A10 | chr2 | 161427044 | 161427345 | 23 |
| . | chr20 | 56925895 | 56926196 | 24 |
| SLC6A15; SLC6A15 | chr12 | 84912868 | 84913169 | 25 |

2. The method of claim 1, wherein the sample is a tissue sample, a blood sample, a stool sample, or a blood product sample.

3. The method of any one of the preceding claims, wherein the sample comprises DNA that is isolated from blood or plasma of the human subject.

4. The method of any one of the preceding claims, wherein the DNA is cell-free DNA (cfDNA) of the human subject.

5. The method of any one of the preceding claims, wherein the method comprises determining the methylation status of each of the one or more markers using next generation sequencing (NGS).

6. The method of any one of the preceding claims, wherein the method comprises using one or more capture baits that enrich for a target region to capture one or more corresponding methylation locus / loci.

7. The method of any one of the preceding claims, wherein each methylation locus is equal to or less than 302bp in length.

8. A method of any one of the preceding claims, the method comprising:
converting unmethylated cytosines of a plurality of DNA fragments in a sample into uracils to generate a plurality of converted DNA fragments, wherein the plurality of DNA fragments were obtained from a biological sample; and sequencing the plurality of converted DNA fragments to generate a plurality of sequence reads, wherein each sequence read corresponds to a converted DNA fragment.

9. The method of claim 8, wherein the plurality of DNA fragments (in total) comprise at least 1 ng, at least 5 ng, at least 10 ng, or at least 20 ng of DNA.

10. The method of claim 8 or 9, wherein the plurality of DNA fragments consist essentially of DNA fragments each of which has a length in a range from 1 00bp to 600bp (e.g., from about 125bp to about 200bp, or from about 140bp to about 160bp (e.g., for cfDNA))(e.g., about 150bp to about 350bp, or from about 200bp to about 300bp (e.g., for sheared DNA)).

11. The method of claim 8 or 9, wherein the plurality of DNA fragments consist essentially of DNA fragments each of which has a length in a range from 1000bp to 200,000bp.

12. The method of claim 8, wherein each of the plurality of sequence reads are at least 50bp, at least 100bp, at least 150bp, at least 200bp, at least 300bp, or more.

**FIG. 1** – *DNA methylation in normal vs. cancer cells*

**FIG. 2** - *Schematic workflow for markers discovery*

FIG. 3 – *Hierarchical clustering of discovered markers from PAAD-TCGA*

GO: Biological Process

embryonic morphogenesis (n = 12)

tube morphogenesis (n = 7)

regulation of transcription from RNA polymerase II promoter (n = 12)

tube development (n = 7)

tissue morphogenesis (n = 6)

positive regulation of transcription from RNA polymerase II promoter (n = 8)

regulation of transcription (n = 21)

heart morphogenesis (n = 4)

palate development (n = 3)

morphogenesis in epithelium (n = 5)

GO: Molecular Function

transcription factor activity (n = 25)

sequence-specific DNA binding (n = 17)

transcription regulator activity (n = 21)

DNA binding (n = 21)

potassium channel activity (n = 2)

protein heterodimerization activity (n = 4)

**FIG. 4** – *GOs Biological process and Molecular function analysis*

**Pathways**

Transcriptional Regulatory Network in Embryonic Stem Cells (n = 5)

Wnt/_ -catening Signaling (n = 6)

−log10(FDR)

**FIG. 5** – *Pathway analysis*

**FIG. 6** - *Kaplan-Meier analysis on hierarchical clustered divided patient groups from TCGA-PAAD*

Europäisches
Patentamt

European
Patent Office

Office européen
des brevets

**EUROPEAN SEARCH REPORT**

Application Number

EP 23 18 0753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/154682 A2 (GRAIL INC [US]) 30 July 2020 (2020-07-30) * sequence listing; claims 1-27,51,64,75-77,102 * * paragraphs [0015], [0019], [0027] - [0029], [0152] - [0156] * * example 4 * | 1-12 | INV. C12Q1/6886 |
| X | US 2014/274748 A1 (AHLQUIST DAVID A [US] ET AL) 18 September 2014 (2014-09-18) * claims 1-45 * * paragraphs [0031] - [0037], [0116] - [0140] * * examples 1-3; tables 1-2 * | 1-12 | |
| X | HAN TIANCHENG ET AL: "An ultrasensitive approach for cancer screening and tissue of origin prediction based on targeted methylation sequencing of cell-free DNA.", JOURNAL OF CLINICAL ONCOLOGY, vol. 40, no. 16_suppl, 1 June 2022 (2022-06-01), pages 10553-10553, XP093102338, US ISSN: 0732-183X, DOI: 10.1200/JCO.2022.40.16_suppl.10553 * the whole document * | 1-12 | |

TECHNICAL FIELDS SEARCHED (IPC)

C12Q

-/--

The present search report has been drawn up for all claims

| Place of search | Date of completion of the search | Examiner |
|---|---|---|
| The Hague | 17 November 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**EUROPEAN SEARCH REPORT**

Application Number

EP 23 18 0753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAOLI YIN ET AL: "Identification of prognosis-related molecular subgroups based on DNA methylation in pancreatic cancer", CLINICAL EPIGENETICS, BIOMED CENTRAL LTD, LONDON, UK, vol. 13, no. 1, 12 May 2021 (2021-05-12), pages 1-16, XP021290733, ISSN: 1868-7075, DOI: 10.1186/S13148-021-01090-W * the whole document * | 1-12 | |
| X | J. B. KISIEL ET AL: "New DNA Methylation Markers for Pancreatic Cancer: Discovery, Tissue Validation, and Pilot Testing in Pancreatic Juice", CLINICAL CANCER RESEARCH, vol. 21, no. 19, 28 May 2015 (2015-05-28), pages 4473-4481, XP055330092, US ISSN: 1078-0432, DOI: 10.1158/1078-0432.CCR-14-2469 * the whole document * | 1-12 | |
| X | JOO MI YI ET AL: "Novel Methylation Biomarker Panel for the Early Detection of Pancreatic Cancer", CLINICAL CANCER RESEARCH, vol. 19, no. 23, 2 October 2013 (2013-10-02), pages 6544-6555, XP055745265, US ISSN: 1078-0432, DOI: 10.1158/1078-0432.CCR-12-3224 * the whole document * | 1-12 | TECHNICAL FIELDS SEARCHED (IPC) |

-/--

The present search report has been drawn up for all claims

| Place of search | Date of completion of the search | Examiner |
|---|---|---|
| The Hague | 17 November 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## EUROPEAN SEARCH REPORT

Application Number

EP 23 18 0753

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANATOLIY A. MELNIKOV ET AL: "Methylation profile of circulating plasma DNA in patients with pancreatic cancer", JOURNAL OF SURGICAL ONCOLOGY, vol. 99, no. 2, 1 February 2009 (2009-02-01), pages 119-122, XP055055128, ISSN: 0022-4790, DOI: 10.1002/jso.21208 * the whole document * | 1-12 | |
| X | WO 2016/205233 A2 (CEPHEID [US]) 22 December 2016 (2016-12-22) * claims 1-122 * * paragraph [534-] * | 1-12 | |
| X | HUANG JINYONG ET AL: "Cancer Detection and Classification by CpG Island Hypermethylation Signatures in Plasma Cell-Free DNA", CANCERS, M D P I AG, CH , vol. 13, no. 22 9 November 2021 (2021-11-09), page 5611, XP009546508, ISSN: 2072-6694, DOI: 10.3390/CANCERS13225611 Retrieved from the Internet: URL:https://www.mdpi.com/2072-6694/13/22/5611 [retrieved on 2021-11-09] * the whole document * * figures 1-5; table 1 * | 1-12 | TECHNICAL FIELDS SEARCHED (IPC) |

-/--

The present search report has been drawn up for all claims

| Place of search | Date of completion of the search | Examiner |
|---|---|---|
| The Hague | 17 November 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 18 0753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAJUMDER SHOUNAK ET AL: "High Detection Rates of Pancreatic Cancer Across Stages by Plasma Assay of Novel Methylated DNA Markers and CA19-9", CLINICAL CANCER RESEARCH, vol. 27, no. 9, 1 May 2021 (2021-05-01), pages 2523-2532, XP093007223, US ISSN: 1078-0432, DOI: 10.1158/1078-0432.CCR-20-0235 Retrieved from the Internet: URL:https://watermark.silverchair.com/2523 .pdf?token=AQECAHi208BE49Ooan9kkhW_Ercy7Dm 3ZL_9Cf3qfKAc485ysgAAAtMwggLPBgkqhkiG9w0BB wgggLAMIICvAIBADCCArUGCSqGSIb3DQEHATAeBgl ghkgBZQMEAS4wEQQMVwTSzhjXhK3XgSLmAgEQgIICh oyPaIK_GHasn3EbfZq7Pxt-xm60z37UzqEom6hfdYS NqRrBlQZrrP_9dbRAlQpu70k5bmJBl30QBhQBLp7CM Tm9H1HF5ou> * the whole document * | 1-12 | |

----- 

TECHNICAL FIELDS SEARCHED (IPC)

The present search report has been drawn up for all claims

| Place of search | Date of completion of the search | Examiner |
|---|---|---|
| The Hague | 17 November 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

## ANNEX TO THE EUROPEAN SEARCH REPORT
## ON EUROPEAN PATENT APPLICATION NO.

EP 23 18 0753

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2023

| Patent document cited in search report | | Publication date | Patent family member(s) | | Publication date |
|---|---|---|---|---|---|
| WO 2020154682 | A2 | 30-07-2020 | AU | 2020212057 A1 | 26-08-2021 |
| | | | CA | 3127762 A1 | 30-07-2020 |
| | | | CN | 113728115 A | 30-11-2021 |
| | | | EP | 3914736 A2 | 01-12-2021 |
| | | | IL | 285126 A | 30-09-2021 |
| | | | US | 2022090207 A1 | 24-03-2022 |
| | | | US | 2022119890 A1 | 21-04-2022 |
| | | | WO | 2020154682 A2 | 30-07-2020 |
| US 2014274748 | A1 | 18-09-2014 | AU | 2014244608 A1 | 10-09-2015 |
| | | | CA | 2902916 A1 | 02-10-2014 |
| | | | CN | 105143465 A | 09-12-2015 |
| | | | EP | 2971179 A2 | 20-01-2016 |
| | | | EP | 3543360 A2 | 25-09-2019 |
| | | | EP | 3878977 A1 | 15-09-2021 |
| | | | EP | 4234721 A2 | 30-08-2023 |
| | | | ES | 2712798 T3 | 14-05-2019 |
| | | | ES | 2859645 T3 | 04-10-2021 |
| | | | ES | 2947873 T3 | 23-08-2023 |
| | | | US | 2014274748 A1 | 18-09-2014 |
| | | | US | 2016040246 A1 | 11-02-2016 |
| | | | US | 2016355892 A1 | 08-12-2016 |
| | | | US | 2018258498 A1 | 13-09-2018 |
| | | | US | 2020283853 A1 | 10-09-2020 |
| | | | WO | 2014159650 A2 | 02-10-2014 |
| | | | WO | 2014159652 A2 | 02-10-2014 |
| WO 2016205233 | A2 | 22-12-2016 | AU | 2016277943 A1 | 01-02-2018 |
| | | | AU | 2022279465 A1 | 02-02-2023 |
| | | | CA | 2989573 A1 | 22-12-2016 |
| | | | CN | 107922941 A | 17-04-2018 |
| | | | EP | 3307885 A2 | 18-04-2018 |
| | | | EP | 3839047 A1 | 23-06-2021 |
| | | | ES | 2842205 T3 | 13-07-2021 |
| | | | JP | 7020922 B2 | 16-02-2022 |
| | | | JP | 2018524978 A | 06-09-2018 |
| | | | JP | 2022070885 A | 13-05-2022 |
| | | | US | 2017137871 A1 | 18-05-2017 |
| | | | WO | 2016205233 A2 | 22-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6265171 B

**Non-patent literature cited in the description**

- **HERMAN et al.** *Proc. Natl. Acad. 20 Sci. USA,* 1992, vol. 93, 9821-9826
- **LIU Y et al.** *Nucleic Acids Res.,* 07 April 2017, vol. 45 (6), e39
- **HUSSMANN D ; HANSEN LL.** *Methods Mol Biol.,* 2018, vol. 1708, 551-571
- **BEIKIRCHER et al.** *Methods Mol Biol.,* 2018, vol. 1708, 407-424
- **FROMMER et al.** *Proc Natl Acad Sci U S A.,* 01 March 1992, vol. 89 (5), 1827-31
- **CAMPAN M et al.** *Methods Mol Biol.,* 2018, vol. 1708, 497-513
- **HERMAN et al.** *Proc. Natl. Acad. Sci. USA,* 1996, vol. 93, 9821-9826
- **FACKLER MJ ; SUKUMAR S.** *Methods Mol Biol.,* 2018, vol. 1708, 473-496
- **GONZALGO ML ; LIANG G.** *Nat Protoc.,* 2007, vol. 2 (8), 1931-6
- **HERMAN.** *Proc. Natl. Acad. Sci. USA,* 1992, vol. 93, 9821-9826
- **HUSSMANN.** *Methods Mol Biol.,* 2018, vol. 1708, 551-571
- **FACKLER.** *Methods Mol Biol.,* 2018, vol. 1708, 473-496
- **LIU.** *Nucleic Acids Res.,* 2017, vol. 45 (6), e39
- **GONZALGO.** *Nat Protoc.,* 2007, vol. 2 (8), 1931-6
- **FROMMER.** *Proc Natl Acad Sci U S A. 1,* 1992, vol. 89 (5), 1827-31
- **CAMPAN.** *Methods Mol Biol.,* 2018, vol. 1708, 497-513